(19) **Europäisches Patentamt / European Patent Office / Office européen des brevets**

(11) **EP 4 674 409 A1**

(12) **EUROPEAN PATENT APPLICATION**

(43) Date of publication:
**07.01.2026 Bulletin 2026/02**

(21) Application number: **24306112.4**

(22) Date of filing: **04.07.2024**

(51) International Patent Classification (IPC):
**A61K 9/28** (2006.01)     **A61K 47/32** (2006.01)
**A61K 47/36** (2006.01)     **A61K 47/40** (2006.01)

(52) Cooperative Patent Classification (CPC):
**A61K 9/286; A61K 9/2866; A61K 39/00;**
**A61K 9/205**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **ROQUETTE FRERES**
**62136 Lestrem (FR)**

(72) Inventors:
- **FOO, Wen Chin**
  **138667 SINGAPORE (SG)**
- **CHOW, Keat-Theng**
  **138667 SINGAPORE (SG)**

(74) Representative: **Plasseraud IP**
**104 Rue de Richelieu**
**CS92104**
**75080 Paris Cedex 02 (FR)**

(54) **ORAL TABLETS FOR THE COLONIC DELIVERY OF THERAPEUTIC PROTEINS**

(57) The invention related to an oral colon targeted tablet comprising a tablet core coated with a film-coating composition, said tablet core comprising a powder of a therapeutic protein co-dried with a substituted cyclodextrin, and said film-coating coating composition comprising a water-insoluble polymer and a soluble fiber. It also related to a process for making such tablets, and to the use thereof as a medicament.

**EP 4 674 409 A1**

## Description

### Technical Field

The invention pertains to the field of oral tablets for the colonic delivery of therapeutic proteins such as antibodies.

### Background Art

Colon targeted drug delivery systems have gained a great deal of attention as potential carriers for the local treatment of colonic diseases with reduced systemic side effects and also for the enhanced oral delivery of various therapeutics vulnerable to acidic and enzymatic degradation in the upper gastrointestinal tract. In recent years, the global pharmaceutical market for biologics has grown, and increasing demand for a more patient-friendly drug administration system highlights the importance of colonic drug delivery as a noninvasive delivery approach for macromolecules. Colon-targeted drug delivery systems for macromolecules can provide therapeutic benefits including better patient compliance (because they are pain-free and can be self-administered) and lower costs. Therefore, to achieve more efficient colonic drug delivery for local or systemic drug effects, various strategies have been explored including pH-dependent systems, enzyme-triggered systems, receptor-mediated systems, and magnetically-driven systems.

Another issue arising from these oral delivery systems, in that most of them are tablets, and biologics like proteins don't resist well to the mechanical stress induced by compression, such that part of the activity of the biologic is lost during tablet manufacturing.

Despite the multiple strategies developed, there is still no colon targeted oral dosage forms of biologics on the market.

### Technical Problem

It was thus an object of the present invention to provide a colon targeted oral delivery system for therapeutic proteins such as antibodies.

It was another object of the present invention to provide a colon targeted oral delivery system for therapeutic proteins that can be prepared with minimal loss of activity of the protein.

### Presentation of the Invention

The inventors successfully developed oral tablets that can be used for the colonic delivery of therapeutic proteins, and that can be prepared with minimal loss of protein activity.

These tablets comprise a tablet core comprising a therapeutic protein and a substituted cyclodextrin such as hydroxypropyl-beta-cyclodextrin (HPBCD). The therapeutic protein and the substituted cyclodextrin are co-dried. This tablet core is coated with a film-coating composition, comprising a water-insoluble polymer such as ethylcellulose and a soluble fiber (e.g., NUTRIOSE®).

As it is apparent from the examples herein after, the tablets can be prepared with minimal loss of protein activity (Examples sections B, C, D, F).

Efficient colon targeting is achieved, as the protein's integrity is preserved in the upper GIT. Furthermore, almost no release of the therapeutic protein occurs before reaching the colon. In colonic condition, the protein is advantageously released and biologically active (Examples sections A, G, H, I).

The inventors also showed that local administration of therapeutic proteins at colonic level was efficient, thus supporting that oral administration of therapeutic proteins in diseases such as inflammatory bowel diseases (IBDs) is a relevant route of administration (Examples section J).

Additionally, it was found that the use of a substituted cyclodextrin such as HPBCD in the tablet core may further improve the treatment efficiency (Examples sections J-III and J-IV).

### Brief Description of the Invention

The invention first relates to an oral colon targeted tablet comprising a tablet core coated with a film-coating composition, said tablet core comprising a therapeutic protein powder, said therapeutic protein powder being obtained by drying a liquid comprising a therapeutic protein and a substituted cyclodextrin, and said film-coating coating composition comprising a water-insoluble polymer and a soluble fiber.

Preferably, said substituted cyclodextrin is substituted with alkyl groups and/or hydroxyalkyl groups and/or sulfoalkyl group. Preferably, said substituted cyclodextrin is substituted with alkyl groups and/or hydroxyalkyl groups and/or sulfoalkyl group having 1 to 5 carbon atoms. Preferably, said substituted cyclodextrin is selected from hydroxypropyl-cyclodextrin, methyl-cyclodextrin, sulfobutyl ether cyclodextrin, or from a mixture thereof. It is even more preferably hydroxypropyl-beta-cyclodextrin (HPBCD).

Preferably, the therapeutic protein is an antibody. Preferably, the therapeutic protein is a TNF-α inhibitor.

Preferably, the water-insoluble polymer is selected from ethylcellulose, cellulose derivatives, acrylic and/or methacrylic ester polymers, polymers or copolymers of acrylate or methacrylate polyvinyl esters, starch derivatives, polyvinyl acetates, polyacrylic acid esters, butadiene styrene copolymers methacrylate ester copolymers, cellulose acetate phtalate, polyvinyl acetate phtalate, shellac, methacrylic acid copolymers, cellulose acetate trimellitate, hydroxypropyl methylcellulose phtalate, zein, starch acetate, or from any mixture thereof. It is even more preferably ethylcellulose.

Preferably, the soluble fiber is selected from xylooligosaccharides, inulin, oligofructoses, fructo-oligosaccharides (FOS), lactulose, galactomannan, suitable hydrolysates thereof, indigestible polydextrose, indigestible dextrins, partial hydrolysates thereof, trans-galacto-oligosaccharides (GOS), xylo-oligosaccharides (XOS), acemannans, lentinans, beta-glucans, partial hydrolysates thereof, polysaccharides-K (PSK), indigestible maltodextrins, partial hydrolysates thereof, or from any mixture thereof. It is even more preferably selected from a soluble indigestible maltodextrin, a soluble indigestible dextrin, or from a mixture thereof.

Preferably, the water-insoluble polymer:soluble fiber dry weight ratio in the film-coating composition is selected from (1:1) to (8:1).

The invention also relates to a process form making such oral colon targeted tablet comprising:

- a step (a) of obtaining said tablet core; and,
- a step (b) of coating said tablet core with said film-coating composition.

The invention also relates to an oral colon targeted tablet obtainable from such process.

The invention also relates to the use of the tablets described herein as a medicament, preferably for the treatment of an inflammatory bowel disease.

## Brief Description of Drawings

Other features, details and advantages will be shown in the following detailed description and on the figures, on which:

### Fig. 1

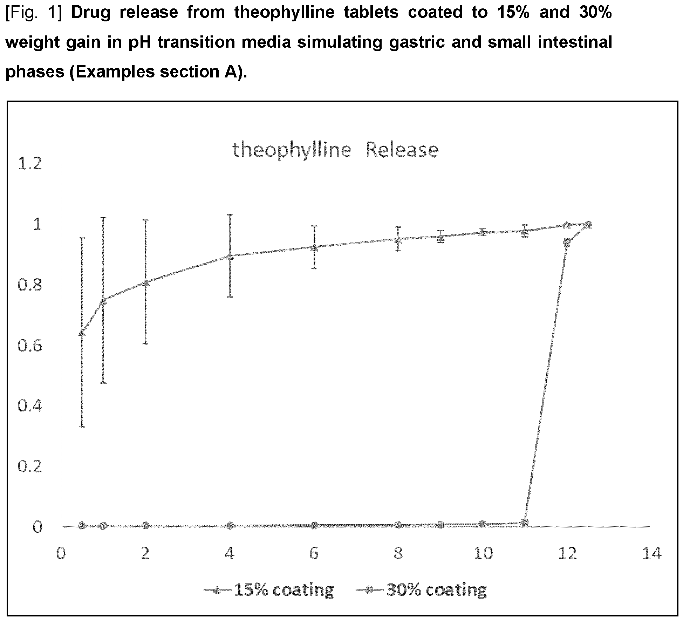

[Fig. 1] (Examples section A) is a curve showing drug release from theophylline tablets coated to 15% and 30% coating in pH transition media simulating gastric and small intestinal phases.

### Fig. 2

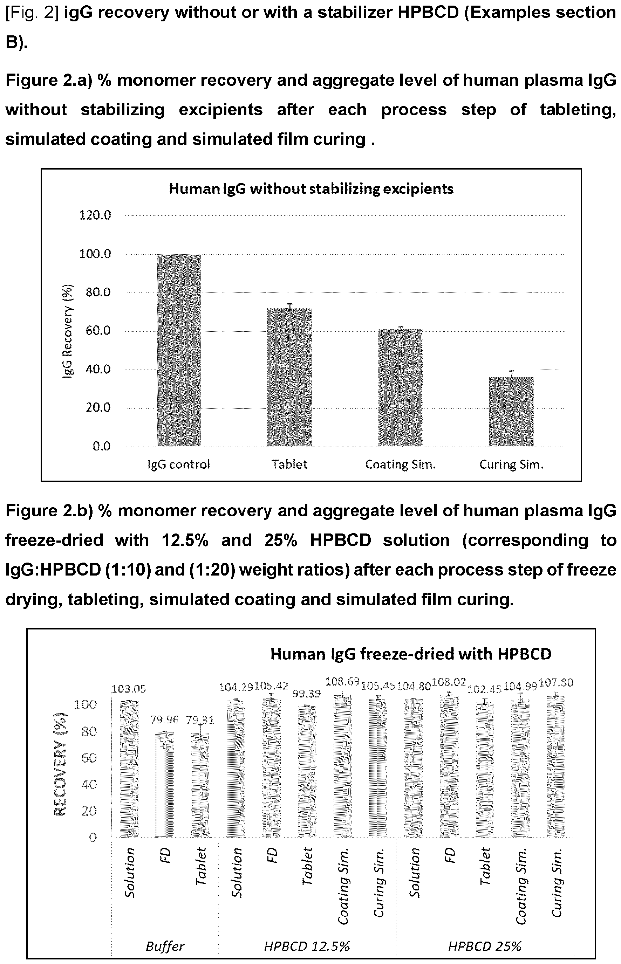

[Fig. 2.a] (Examples section B) is a bar chart showing the % monomer recovery and aggregate level of human plasma IgG without stabilizing excipients after each process step of tableting, simulated coating and simulated film curing.
[Fig. 2.b] is a bar chart showing the % monomer recovery and aggregate level of human plasma IgG freeze-dried with 12.5% and 25% HPBCD solution (corresponding to IgG:HPBCD (1:10) and (1:20) weight ratios), after each process step of freeze drying, tableting, simulated coating and simulated film curing.

### Fig. 3

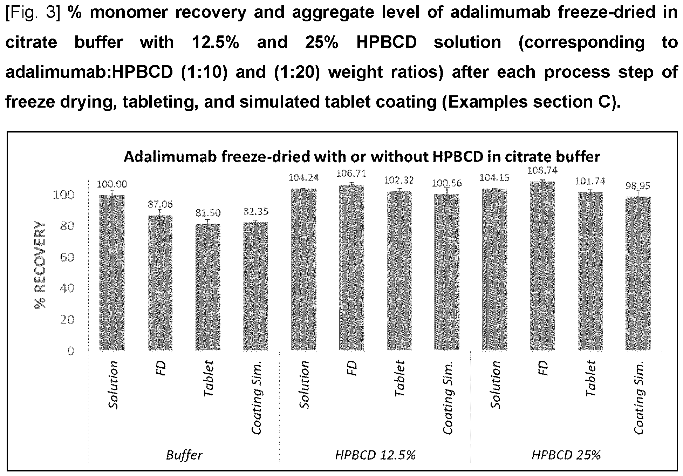

[Fig. 3] (Examples section C) is a bar chart showing the monomer recovery and aggregate level of adalimumab freeze-dried in citrate buffer with 12.5% and 25% HPBCD solution (corresponding to adalimumab:HPBCD (1:10) and (1:20) weight ratios) after each process step of freeze drying, tableting, and simulated tablet coating.

### Fig. 4

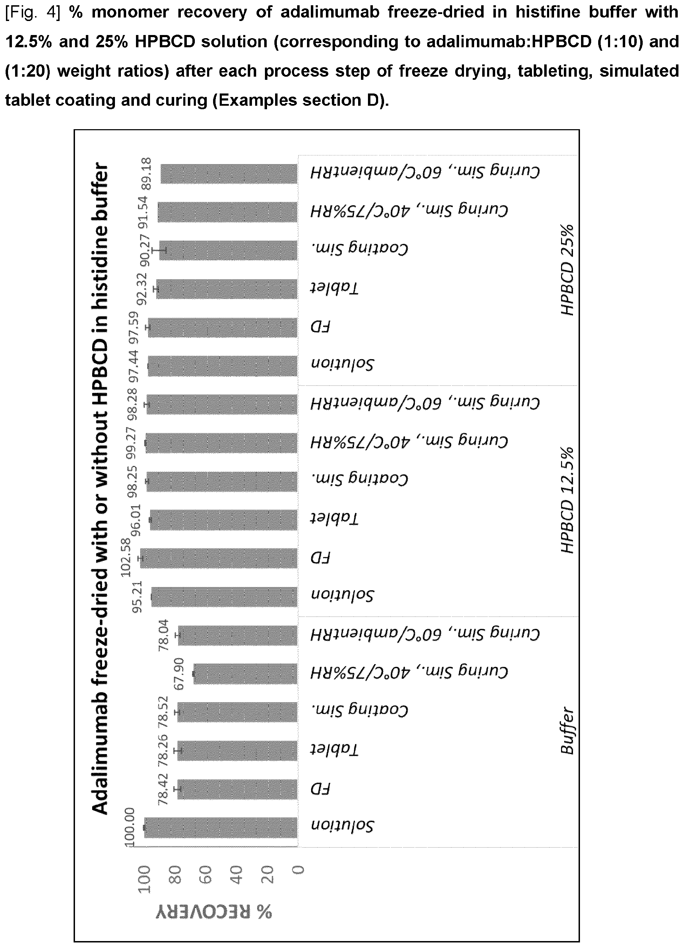

[Fig. 4] (Examples section D) is a bar chart showing the % monomer recovery of adalimumab freeze-dried in histidine buffer with 12.5% and 25% HPBCD solution (corresponding to adalimumab:HPBCD (1:10) and (1:20) weight ratios) after each process step of freeze drying, tableting, simulated tablet coating and curing.

### Fig. 5

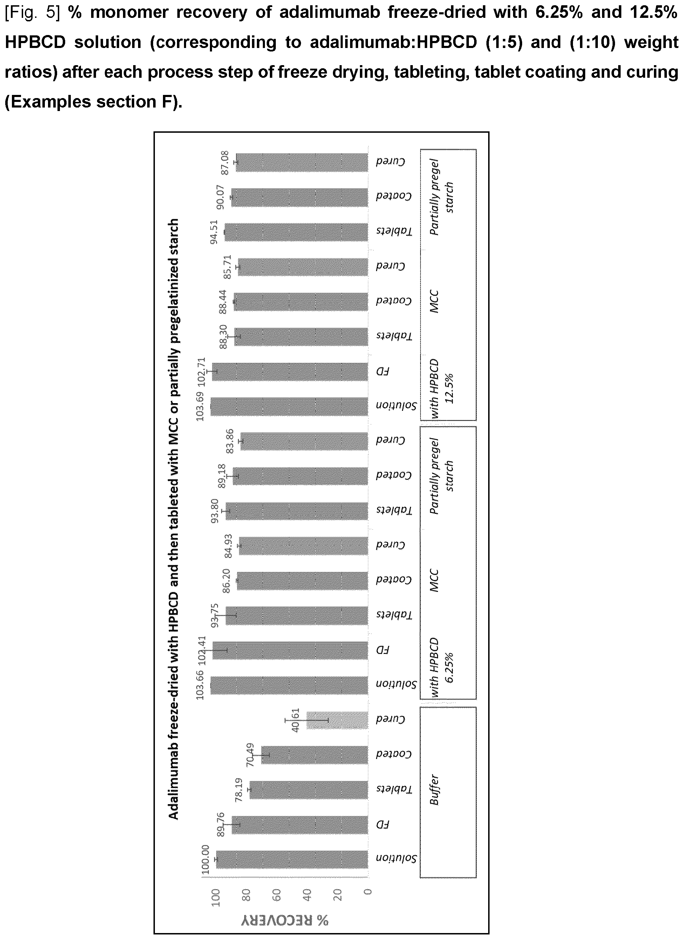

[Fig. 5] (Examples section F) is a bar chart showing the % monomer recovery of adalimumab freeze-dried with 6.25% and 12.5% HPBCD solution (corresponding to adalimumab:HPBCD (1:5) and (1:10) weight ratios) after each process step of freeze drying, tableting, tablet coating and curing.

### Fig. 6

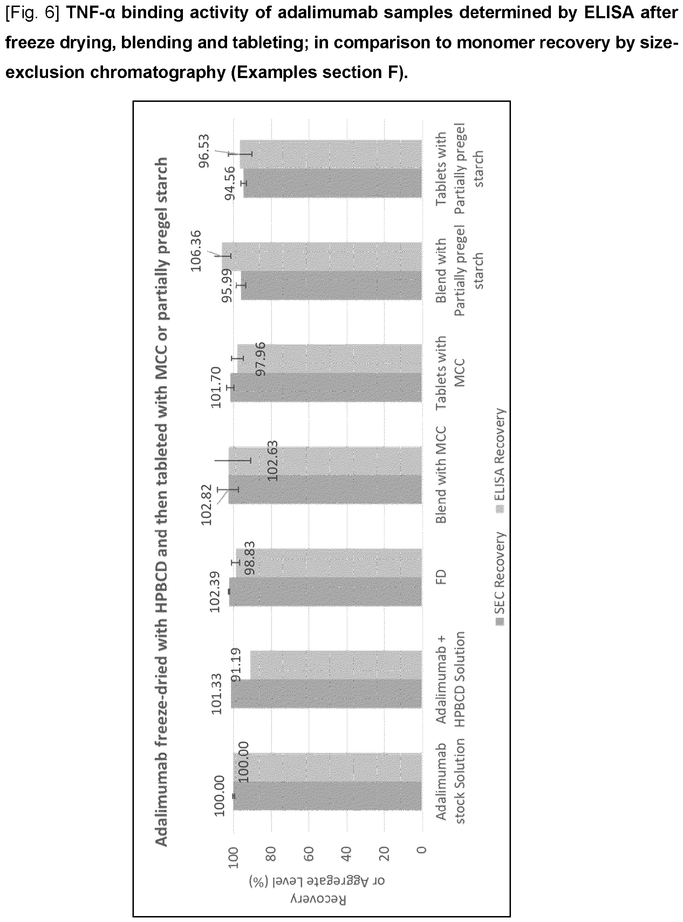

[Fig. 6] (Examples section F) is a bar chart showing the TNF-$\alpha$ binding activity of adalimumab samples determined by ELISA after freeze drying, blending and tableting; in comparison to monomer recovery by size-exclusion chromatography.

**Fig. 7**

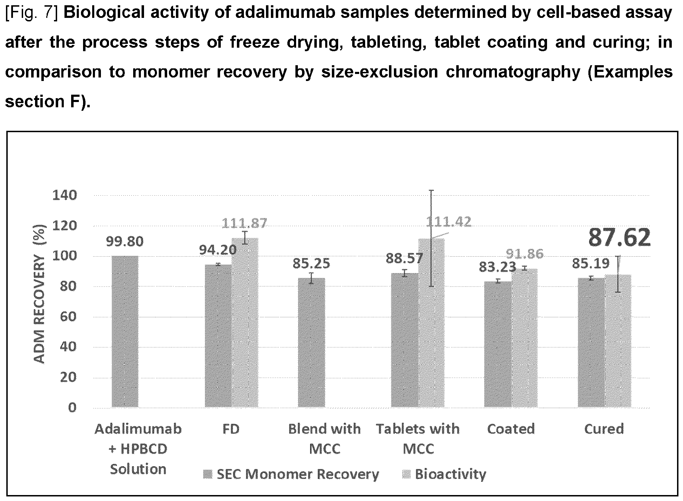

[Fig. 7] (Examples section F) is a bar chart showing the biological activity of adalimumab samples determined by cell-based assay after the process steps of freeze drying, tableting, tablet coating and curing; in comparison to monomer recovery by size-exclusion chromatography.

**Fig. 8**

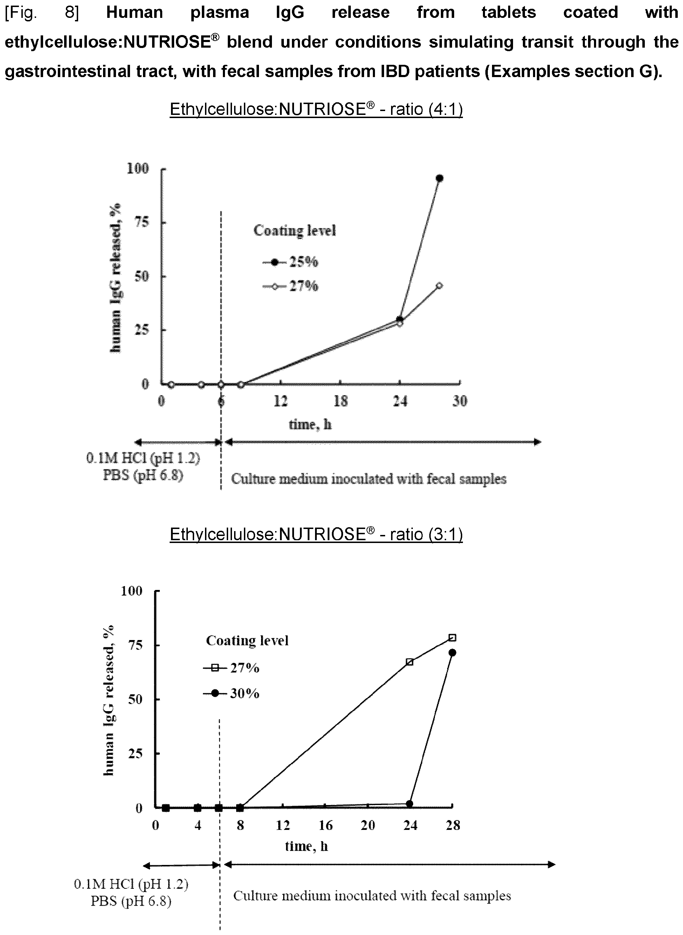

[Fig. 8] (Examples section G) is curves showing human plasma IgG release from tablets coated with Ethylcellulose:NUTRIOSE® blend under conditions simulating transit through the gastrointestinal tract, with fecal samples from IBD patients.

**Fig. 9**

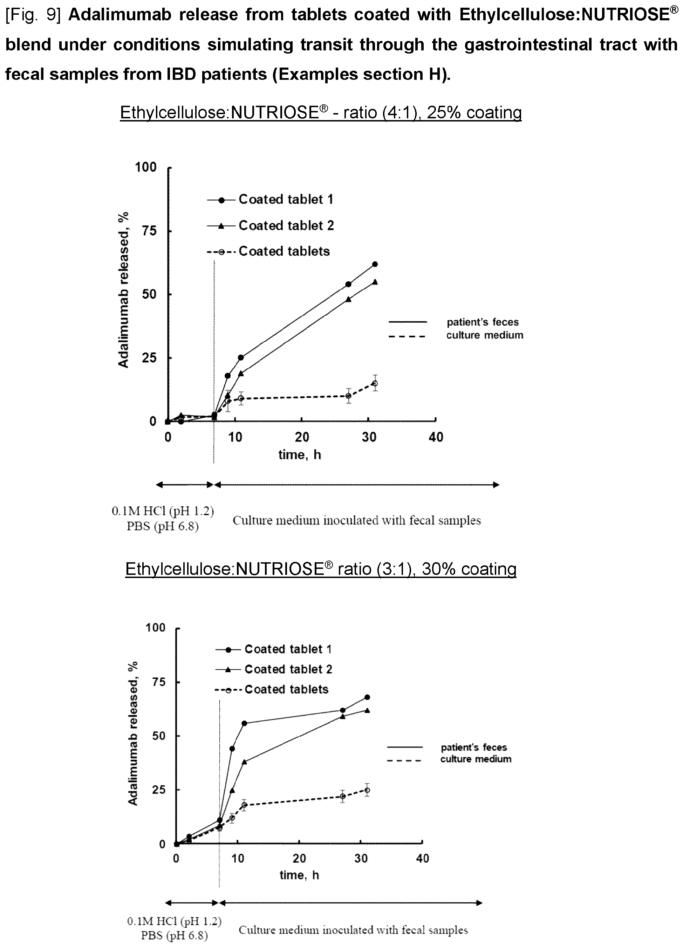

[Fig. 9] (Examples section H) is curves showing adalimumab release from tablets coated with Ethylcellulose:NUTRIOSE® blend under conditions simulating transit through the gastrointestinal tract with fecal samples from IBD patients.

**Fig. 10**

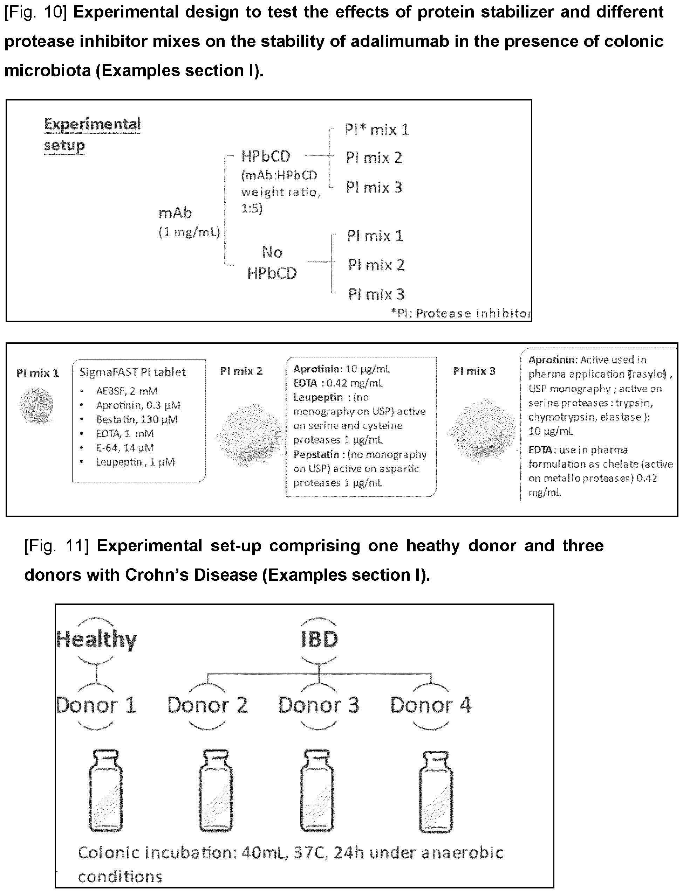

[Fig. 10] is a scheme showing the experimental design of Examples section I, to test the effects of protein stabilizer and different protease inhibitor mixes on the stability of adalimumab in the presence of colonic microbiota.

**Fig. 11**

[Fig. 11] is a scheme showing the experimental set-up of Examples section I, comprising one heathy donor and three donors with Crohn's Disease.

**Fig. 12**

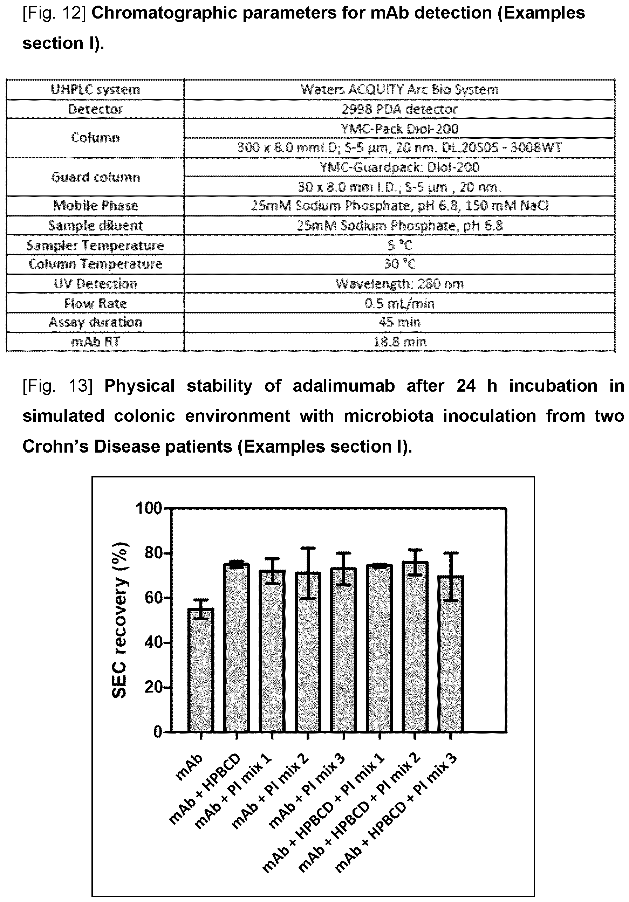

[Fig. 12] is a table showing the chromatographic parameters used in Examples section I for mAb detection.

**Fig. 13**

[Fig. 13] (Examples section I) is a bar chart showing the physical stability of adalimumab after 24 h incubation in simulated colonic environment with microbiota inoculation from two Crohn's Disease patients.

**Fig. 14**

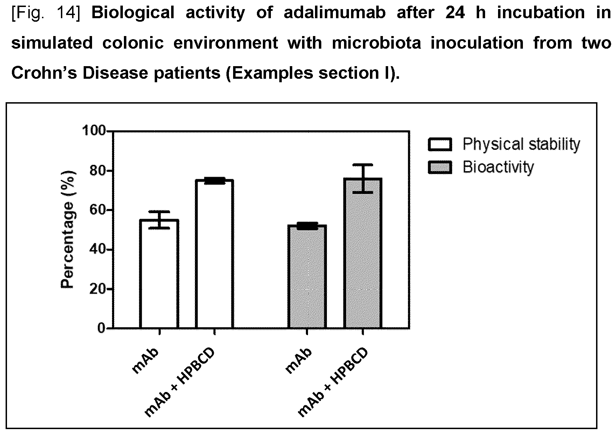

[Fig. 14] (Examples section I) is a bar chart showing the biological activity of adalimumab after 24 h incubation in simulated colonic environment with microbiota inoculation from two Crohn's Disease patients.

**Fig. 15**

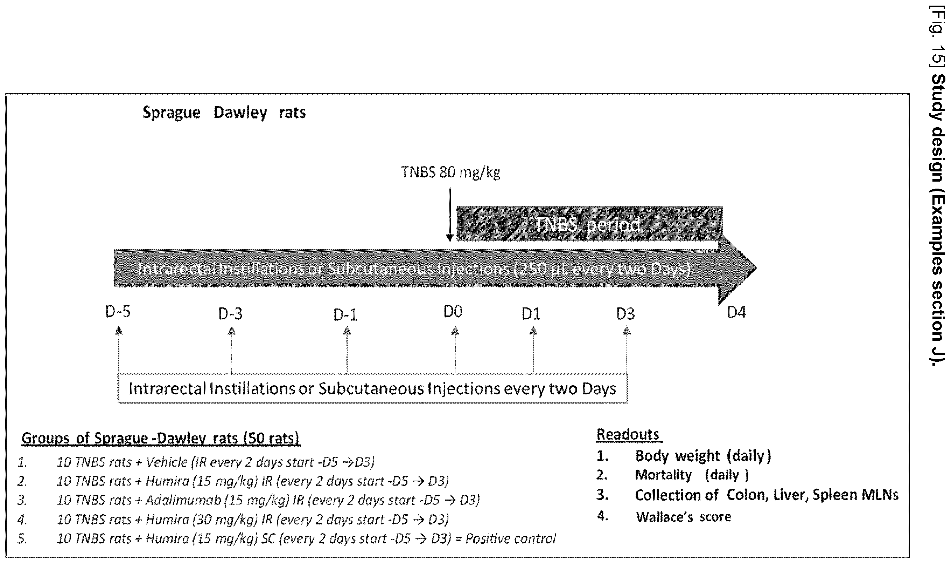

[Fig. 15] is a scheme showing the study design of Examples section J.

**Fig. 16**

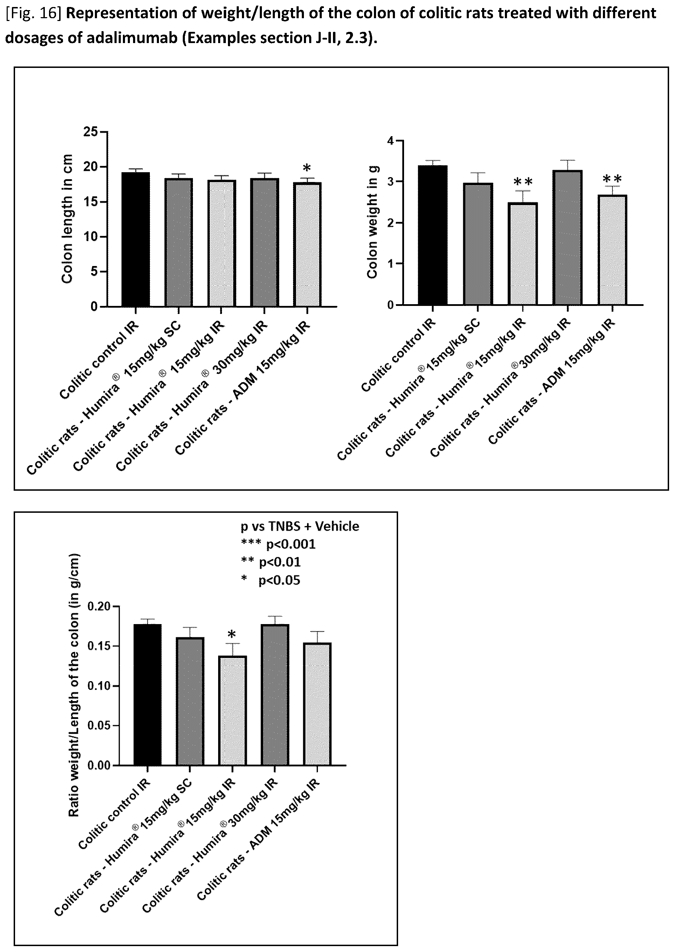

[Fig. 16] (Examples section J-II, 2.3) is bar charts showing the of weight and length of the colon of colitic rats treated with adalimumab (made in-house or commercial (HUMIRA®))

**Fig. 17**

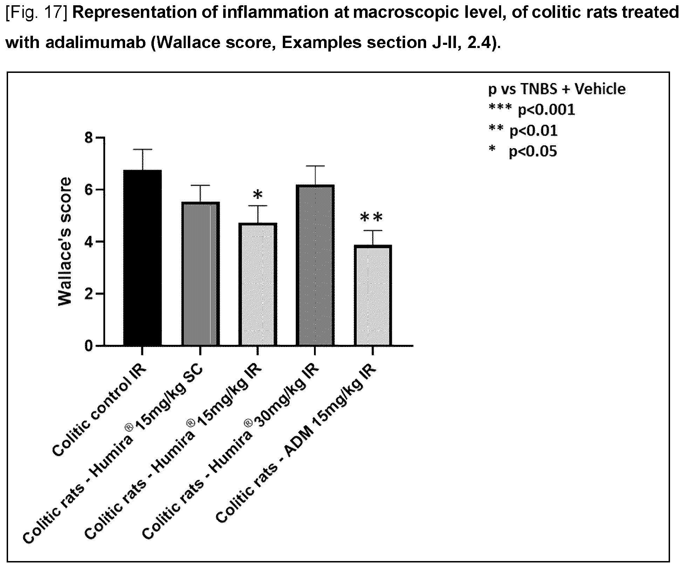

[Fig. 17] (Examples section J-II, 2.4) is a bar chart showing the inflammation at macroscopic level (Wallace score), of colitic rats treated with adalimumab (made in-house or commercial (HUMIRA®))

**Fig. 18**

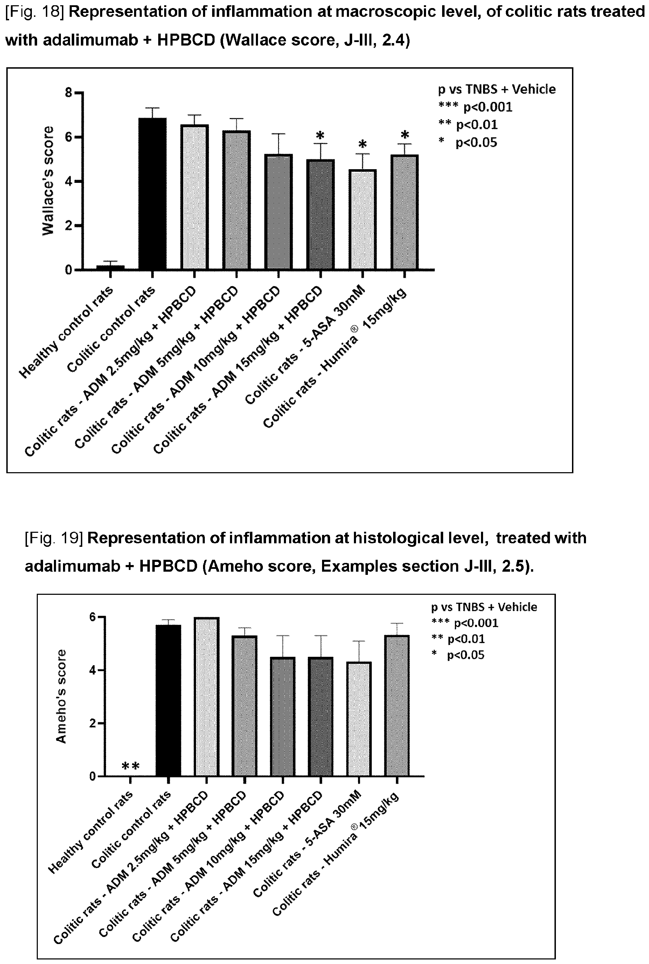

[Fig. 18] (Examples section J-III, 2.4) is a bar chart showing the inflammation at macroscopic level (Wallace score), of colitic rats treated with adalimumab + HPBCD.

**Fig. 19**

[Fig. 19] (Examples section J-III, 2.5) is a bar chart showing the inflammation at histological level (Ameho score), of colitic rats treated with adalimumab + HPBCD.

**Fig. 20**

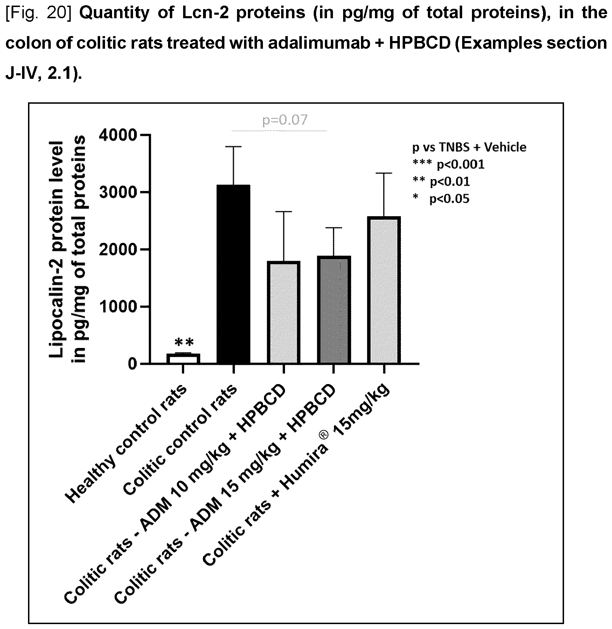

[Fig. 20] (Examples section J-IV, 2.1) is a bar chart showing the quantity of Lcn-2 proteins (in pg/mg of total proteins), in the colon of colitic rats treated with adalimumab + HPBCD.

**Fig. 21**

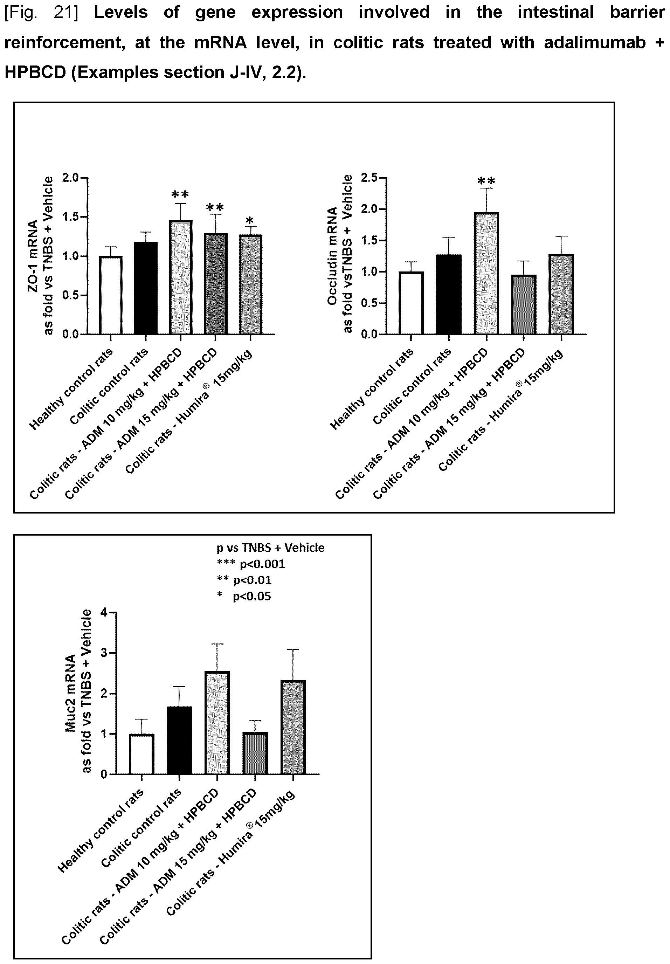

[Fig. 21] (Examples section J-IV, 2.2) is bar charts showing the levels of gene expression involved in the intestinal barrier reinforcement, at the mRNA level, in colitic rats treated with adalimumab + HPBCD.

**Fig. 22**

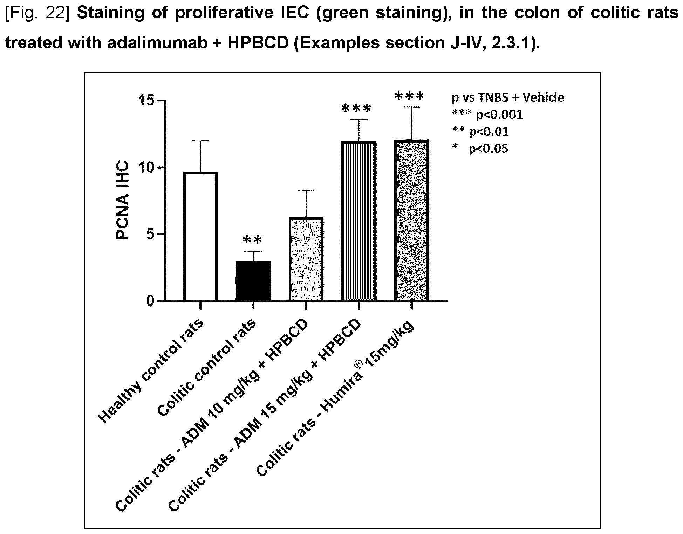

[Fig. 22] (Examples section J-IV, 2.3.1) is bar charts showing the staining of proliferative IEC (green staining), in the colon colitic rats treated with adalimumab + HPBCD.

**Fig. 23**

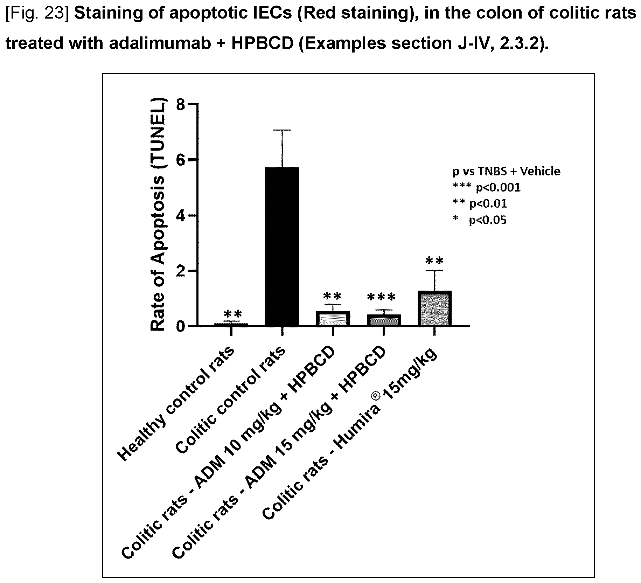

[Fig. 23] (Examples section J-IV, 2.3.2) is bar charts showing the staining of apoptotic IECs (Red staining), in the colon of colitic rats treated with adalimumab + HPBCD.

**Description of Embodiments**

**Oral Colon Targeted tablet**

The invention first relates to an oral colon targeted tablet comprising a tablet core coated with a film-coating composition, said tablet core comprising a therapeutic protein powder, said therapeutic protein powder being obtained by drying a liquid comprising a therapeutic protein and a substituted cyclodextrin, and said film-coating coating composition comprising a water-insoluble polymer and a soluble fiber.

The term "tablet" classically refers to a solid preparation comprising a core obtained by tableting a powder composition directly (direct compression) or after a wet or dry granulation step. The tablet core may be coated or not. In the present disclosure, tablet cores are coated with at least one film-coating composition comprising a water-insoluble polymer and a soluble fiber. In the present disclosure, the tablet core is preferably obtained by direct compression.

The term "oral colon targeted tablet" classically refers to a tablet comprising an active ingredient that targets the colon, and that is bioavailable when taken orally. In the case where the active ingredient is a protein, this mean in particular that at least 50 % of the active ingredient is released in colonic environment. This ability to be released in colonic conditions when taken orally may be determined by the person skilled in the art by an *in vitro* test measuring the quantity of therapeutic protein released in simulated gastric fluid (SGF), simulated intestinal fluid (SIF), and simulated colonic environment (SCE), as follows: tablets are placed in 0.1 M HCl solution prepared in purified water (SGF) for 1 hour, which is then completely replaced by pH 6.8 phosphate buffer for 5 hours (SIF); then the tablets are exposed to the feces of patients, preferably taken from patient suffering from colonic disease such as IBD, and incubated under anaerobic conditions for 24 hours (SCE).

The % of protein released is preferably a % of active protein and may be determined by any appropriate *in vitro* assay capable of testing the activity of the protein. Such assay may be for example an assay testing the binding-capacity of the protein (e.g., ELISA assay), and/or an assay measuring the protein (structural) integrity, for example by measuring the monomer content of the protein (e.g., size-exclusion chromatography). Another example would be an assay measuring the pharmacological or biological effect of the protein by techniques including western blot or chromatography.

This ability to be released in colonic conditions when taken orally may be determined according to the protocol given in the Example section G-II.

According to this test, the quantity of protein released after 28 hours (1 hour in SGF + 5 hours in SIF + 20 hours in SCE) is preferably equal to or higher than 45%, preferably equal to or higher than 50%, preferably equal to or higher than 55%, preferably equal to or higher than 60%, preferably equal to or higher than 70%, preferably equal to or higher than 75%, preferably equal to or higher than 80%, preferably equal to or higher than 90%, preferably equal to 100%..

Still according to this test, the quantity of protein released in upper GIT (1 hour in SGF + 5 hours in SIF) is preferably equal to or lower than 20%, preferably equal to or lower than 15%, preferably equal to or lower than 10%, preferably equal to or lower than 9%, preferably equal to or lower than 8%, preferably equal to or lower than 7%, preferably equal to or lower than 6%, preferably equal to or lower than 5%, preferably equal to or lower than 4%, preferably equal to or lower than 3%, preferably equal to or lower than 2%, preferably equal to or lower than 1%, preferably equal to 0%.

The tablet core according to the disclosure comprises a therapeutic protein powder that is obtained by drying a liquid comprising a therapeutic protein and a substituted cyclodextrin.

Said liquid may be a solution, a suspension, or a dispersion. It is preferably an aqueous solution, an aqueous suspension, or an aqueous dispersion. It is typically an aqueous solution comprising the therapeutic protein and the substituted cyclodextrin. The drying may be performed by freeze-drying, spray-drying, spray-freeze drying, electrospray, electrospray drying, vacuum drying, or thin film freeze drying. It is preferably performed by freeze-drying.

The terms "therapeutic protein" herein refers to any protein of pharmaceutical, veterinary or nutraceutical interest. Preferably, the therapeutic protein according to the disclosure is a pharmaceutical or veterinary active protein, more preferable a pharmaceutical active protein. The term "protein" classically refers, int its broad meaning, to oligopeptides, peptides and proteins, regardless the way they are manufactured and regardless their number of subunits. They may be native proteins, recombinant proteins, or fusion proteins. They may be functionalized, for example PEGylated. Although they are preferably not functionalized in the present disclosure.

Therapeutic proteins may be selected for example from enzymes, cytokines, hormones, growth factors, plasmatic factors, vaccines, or antibodies. The term "antibodies" encompasses full antibodies or fragments thereof. It may be a human, humanized, animal or chimeric antibody.

Preferably, the therapeutic protein according to the disclosure is an antibody, more preferably a human, humanized, or chimeric antibody. Preferably, the antibody according to the disclosure comprises a Fab region and a Fc region. More preferably, the antibody according to the disclosure has two heavy chains and two light chains. Preferably, the heavy chains have 1 variable domain, and 3 constant domains. Preferably, the lights chains have 1 variable domain, and 1 constant domain. It is thus preferably an IgG.

In a preferred embodiment, the therapeutic protein according to the disclosure is a TNF-$\alpha$ inhibitor, for example selected from golimumab, certolizumab, etanercept, adalimumab, or infliximab. It is preferably selected from antibodies, preferably selected from certolizumab, golimumab, adalimumab, or infliximab. It is more preferably selected from golimumab, adalimumab, or infliximab. It is more preferably selected from golimumab or adalimumab. It is more preferably adalimumab.

Preferably, the therapeutic protein according to the disclosure has a molecular weight equal to or higher than 500 Da, preferably equal to or higher than 1 kDa, preferably equal to or higher than 5 kDa, preferably equal to or higher than 10 kDa, preferably equal to or higher than 50 kDa, preferably equal to or higher than 60 kDa, preferably equal to or higher than 70 kDa, preferably equal to or higher than 80 kDa, preferably equal to or higher than 90 kDa, preferably equal to or higher than 100 kDa, preferably equal to or higher than 110 kDa, preferably equal to or higher than 120 kDa, preferably equal to or higher than 130 kDa, preferably equal to or higher than 140 kDa. It is preferably equal to or lower than 500 kDa, preferably equal to or lower than 400 kDa, preferably equal to or lower than 300 kDa, preferably equal to or lower than 200 kDa, preferably equal to or lower than 190 kDa, preferably equal to or lower than 180 kDa, preferably equal to or lower than 170 kDa, preferably equal to or lower than 160 kDa, preferably equal to or lower than 150 kDa.

Preferably, the amount of therapeutic protein, as dry weight, in the tablet core according to the disclosure is equal to or higher than 1 %, preferably equal to or higher than 2%, preferably equal to or higher than 3%, preferably equal to or higher than 4%, preferably equal to or higher than 5%, preferably equal to or higher than 6%, preferably equal to or higher than 7%; said percentage being expressed with respect to the total dry weight of said tablet core. This amount is in general equal to or lower than 30%, even equal to or lower than 25%, even equal to or lower than 20%, even equal to or lower than 15%, even equal to or lower than 14%, even equal to or lower than 13%, even equal to or lower than 12%, even equal to or lower than 11%, even equal to or lower than 10%, even equal to or lower than 9%. It is for example equal to 8%.

The therapeutic protein powder according to the disclosure also comprises a substituted cyclodextrin.

The expression « substituted cyclodextrin» classically refers to a mixture of cyclodextrin molecules, further comprising the residual substances resulting from its manufacturing process. Contrary to chemical substances with well-defined structure, substituted cyclodextrins generally are a mixture of substituted cyclodextrin molecules having different substitution patterns, and which are thus structurally different. It is understood that a substituted cyclodextrin is different from a cyclodextrin polymer, the latter being composed of cyclodextrin molecules covalently bound to one another.

Preferably, the substituted cyclodextrin is substituted with alkyl groups and/or hydroxyalkyl groups and/or sulfoalkyl group, preferably having 1 to 5 carbon atoms, preferably 1 to 4, preferably 1 to 3, preferably 1 or 3. Preferably, the substituted cyclodextrin according to the disclosure is substituted by etherification. It is preferably selected from hydroxypropyl-cyclodextrin, methyl-cyclodextrin, sulfobutyl ether cyclodextrin, or from a mixture thereof. It is preferably selected from hydroxypropyl-cyclodextrin, methyl-cyclodextrin, or from a mixture thereof. It is preferably hydroxypropyl-cyclodextrin, more preferably hydroxypropyl-beta-cyclodextrin (HPBCD).

Preferably, the substituted cyclodextrin according to the disclosure, preferably HPBCD, has an average molar substitution degree (MS) equal to or higher than 0.10, preferably equal to or higher than 0.20, preferably equal to or higher than 0.30, preferably equal to or higher than 0.40, preferably equal to or higher than 0.50, preferably equal to or higher than 0.55, preferably equal to or higher than 0.58. Preferably, this MS is equal to or lower than 1.50, preferably equal to or lower than 1.40, preferably equal to or lower than 1.30, preferably equal to or lower than 1.20, preferably equal to or lower than 1.10, preferably equal to or lower than 1.00, preferably equal to or lower than 0.99, preferably equal to or lower than 0.90, preferably equal to or lower than 0.80, preferably equal to or lower than 0.70, preferably equal to or lower than 0.68.

It is reminded that the "average molar substitution degree (MS)" refers to the average number of added substituents per anhydroglucose unit. It should be noted that this MS is different from the average molecular substitution degree (DS), which refers to the average number of added substituents per cyclodextrin molecule, and which is, as a consequence, function of the number of anhydroglucose units forming the original cyclodextrin. For instance, for alkylated β-cyclodextrins, this DS is equal to 7 times the MS, as the β-cyclodextrins are made of 7 anhydroglucose units.

The MS of the hydroxypropyl-cyclodextrin according to the disclosure (e.g., HPBCD), can classically be determined by the person skilled in the art by proton nuclear magnetic resonance (NMR), preferably according to the USP 41 NF 36 method « Hydroxypropyl Betadex ; Molar substitution », as in force on September 1st, 2023.

Preferably, the hydroxypropyl-cyclodextrin according to the disclosure (e.g., HPBCD), has the following substitution profile, as determined by electrospray ionization - Mass spectrometry (ESI-MS):

- signal corresponding to unsubstituted cyclodextrin and hydroxypropyl-cyclodextrin molecules having 1 substitution (HP≤1): equal to or lower than 5 %, preferably equal to or lower than 4 %, preferably equal to or lower than 3 %, preferably equal to or lower than 2 %, preferably equal to or lower than 1 %; and/or,
- signal corresponding to the hydroxypropyl-cyclodextrin molecules having 2 substitutions (HP2): equal to or lower than 5 %, preferably equal to or lower than 4 %, preferably equal to or lower than 3 %, preferably equal to or lower than 2 %; and/or,
- signal corresponding to the hydroxypropyl-cyclodextrin molecules having 3 substitutions (HP3): equal to or lower than 15 %, preferably from 1 to 10 %, preferably from 1 to 8 %, preferably from 2 to 6 %; and/or,
- signal corresponding to the hydroxypropyl-cyclodextrin molecules having 4 substitutions (HP4): equal to or lower than 25 %, preferably from 2 to 20 %, preferably from 4 to 15 %, preferably from 7 to 13 %; and/or,
- signal corresponding to the hydroxypropyl-cyclodextrin molecules having 5 substitutions (HP5): equal to or lower than 40 %, preferably from 1 to 40 %, preferably from 5 to 35 %, preferably from 10 to 30 %, preferably from 15 to 25 %; and/or,
- signal corresponding to the hydroxypropyl-cyclodextrin molecules having 6 substitutions (HP6): equal to or lower than 50 %, preferably from 5 to 50 %, preferably from 10 to 40 %, preferably from 15 to 35 %, preferably from 20 to 30 %; and/or,
- signal corresponding to the hydroxypropyl-cyclodextrin molecules having 7 substitutions (HP7): from 5 to 50 %, preferably from 10 to 40 %, preferably from 15 to 35 %, preferably from 20 to 30 %; and/or,
- signal corresponding to the hydroxypropyl-cyclodextrin molecules having 8 substitutions (HP8): from 1 to 50 %, preferably from 2 to 45 %, preferably from 2 to 30 %, preferably from 2 to 25 %, preferably from 5 to 20 %, preferably from 8 to 15 %; and/or,
- signal corresponding to the hydroxypropyl-cyclodextrin molecules having 9 substitutions (HP9): equal to or lower than 30 %, preferably from 1 to 25 %, preferably from 1 to 10 %, preferably from 1 to 8 %, preferably from 2 to 5 %; and/or,
- signal corresponding to the hydroxypropyl-cyclodextrin molecules having at least 10 substitutions (HP≥10): equal to or lower than 20 %, preferably equal to or lower than 15 %, preferably equal to or lower than 10 %, preferably equal to or lower than 5 %, preferably equal to or lower than 4 %, preferably equal to or lower than 3 %, preferably equal to or lower than 2 %, preferably equal to or lower than 1 %;

these percentages being expressed with respect of the sum of the signals obtained for each substitution for which the signal was higher than the background.

It is understood that "signal" refers to the area under the curve of the ion(s) corresponding to the substitutions degree(s) of interest.

In the present disclosure, the substitution profile is determined by ESI-MS, preferably by calculating the average of the measurements performed in triplicate. For determining this substitution profile, it is possible to proceed according to the following method:

- A solution hydroxypropyl-cyclodextrin is prepared at 1 g.L$^{-1}$ (w/v) in methanol: water (50/50, v/v) with 1 mM sodium acetate. Infusion is performed for 1 min at 10 μL/min and data are recorded. In between two following injections, 500 μL of methanol: water (50/50, v/v) are injected to wash the ion source.
- ESI parameters: Spray voltage: 5 kV; Sheath gas: 9; Auxiliary gas: 2; Sweep gas: 0; Spray voltage: 5 kV; Capillary voltage: 23 V; Capillary temperature: 275 °C; Tube lens: 80 V
- MS parameters: Full scan; Scan ranges: 50-2000 m/z; Mass range: normal; Scan rate: enhanced; Data acquisition time: 1 min.
- For each substituted hydroxypropyl-cyclodextrin species (named as HPX, X being the number of hydroxypropyl substitutions), the corresponding ion current (XIC) is integrated and compared to the sum of all HPX ion currents. As the sodium adduct is the most intense hydroxypropyl-cyclodextrin ion (almost 100 times superior to [M+H]$^+$ or [M+NH4]$^+$), its peak area is integrated to estimate the proportion of the corresponding HPX molecule.

Preferably, the substitution pattern of the hydroxypropyl-cyclodextrin according to the disclosure (e.g., HPBCD) is such that:

- the molar proportion of unsubstituted (no-OHP) moities is from 20 to 70%, preferably from 25 to 60%, preferably from 30 to 50%, preferably from 40 to 50%; and/or,
- the molar proportion of C2 substituted (2 OHP) moieties is from 10 to 50%, preferably from 15 to 45%, preferably from 20 to 40%, preferably from 20 to 35%, preferably from 20 to 30%; and/or,

- the molar proportion of C3 substituted (3 OHP) moieties is from 2 to 15%, preferably from 4 to 10%, preferably from 6 to 9%, preferably from 7 to 8%; and/or,
- the molar proportion of C6 substituted (6 OHP) moieties is from 0.5 to 10%, preferably from 1 to 8%, preferably from 2 to 5%, preferably from 3 to 4%; and/or,
- the molar proportion of C2 and C3 substituted (2,3-di-OHP) moieties is from 1 to 20%, preferably from 3 to 15%, preferably from 5 to 15%, preferably from 5 to 12%, preferably from 6 to 11%, preferably from 7 to 10%; and/or,
- the molar proportion of C2 and C6 substituted (2,6-di-OHP) moieties is from 0.5 to 15%, preferably from 1 to 10%, preferably from 2 to 10%, preferably from 2 to 5%; and/or,
- the molar proportion of twice C3 substituted (3,3'-di-OHP) moieties is equal to or lower than 3%, preferably equal to or lower than 2%, preferably equal to or lower than 1.0%, for example from 0.4 to 1.0%, preferably from 0.5 to 0.9%; and/or,
- the molar proportion of C2, C3 and C6 substituted (2,3,6-tri-OHP) moieties is equal to or lower than 10%, preferably equal to or lower than 5%, preferably equal to or lower than 3%, preferably equal to or lower than 2%, preferably equal to or lower than 1.0, for example from 0.5 to 3%, preferably from 0.5 to 2.0%, preferably from 0.5 to 1.5%, preferably from 0.5 to 1.0%; and/or,
- the molar proportion of substitutions corresponding to mono-substitutions is from 40 to 90%, preferably from 50 to 85%, preferably from 60 to 80%, preferably from 70 to 80%; and/or,
- the molar proportion of substitutions corresponding to di-substitutions is from 5 to 50%, preferably from 10 to 45%, preferably from 15 to 40%, preferably from 20 to 35%, preferably from 20 to 30%; and/or,
- the molar proportion of substitutions corresponding to tri-substitutions is from 0.5 to 15%, preferably from 0.5 to 10%, preferably from 1 to 7%, preferably from 1 to 5%, preferably from 1 to 3%, preferably from 1 to 2%; and/or,
- the C2/C6 substitutions molar ratio is from 1.0 to 15.0, preferably from 1.5 to 12.0, preferably from 2.0 to 10.0, preferably from 2.5 to 9.0, preferably from 3.0 to 8.0, preferably from 4.0 to 8.0, preferably from 4.5 to 7.5, preferably from 5.0 to 7.0, preferably from 4.5 to 6.5, preferably; and/or,
- the C2/C3 substitutions molar ratio is from 0.5 to 5.0, preferably from 1.0 to 4.0, preferably from 1.5 to 3.5, preferably from 2.0 to 3.0, preferably from 2.0 to 2.5.

The percentages correspond to the percentages of anhydroglucose units having the type of substitution considered. For example, a 2 OHP value equal to 30.0% means that 30.0 mol% of the anhydroglucose units of the hydroxypropyl-cyclodextrin are substituted by a hydroxypropyl group at the C2 carbon (and have not further substitutions). As a further example, a 3,3'-di-OHP value equal to 0.4% means that 0.4 mol% of the anhydroglucose units of the hydroxypropyl-cyclodextrin are substituted twice at the C3 carbon i.e., that the C3 carbon bears two hydroxypropyl groups (and the anhydroglucose units have not further substitutions). As a last example, a 2,6-di-OHP value equal to 5.0% means that 5.0 mol% of the anhydroglucose units of the hydroxypropyl-cyclodextrin are substituted by a hydroxypropyl group both at the C2 carbon, and at the C6 carbon (and have not further substitutions). For the first example, mention will be made of mono-substitution, whereas mention will be made of di-substitution for the last two examples.

Preferably, the hydroxypropyl-cyclodextrin according to the disclosure comprises less than 5.0% of substitutions other than those listed above, for example 3,6-OHP substitutions, preferably less than 4.0%, preferably less 3.0%, preferably less than 2.0%, preferably less than 1.0%, preferably less than 0.5%. More preferably, the hydroxypropyl-cyclodextrin according to the disclosure comprises no other types of substitutions than those listed above. The expression "no other types of substitutions" is understood to mean that the anhydroglucose units comprising such substitutions are not detectable, in particular by the "Hakomori"-like method, said method comprising subjecting the hydroxypropyl-cyclodextrin to the following successive steps: permethylation, hydrolysis, reduction, peracetylation.

Indeed, these substitution patterns may be determined by those skilled in the art according to a method analogous to the "Hakomori" method, typically by subjecting the hydroxypropyl-cyclodextrin to the following successive steps: permethylation, hydrolysis, reduction, peracetylation.

It is possible for example to follow the method as described below:

60 % NaH powder (35 mg) is washed with 2 mL of Heptane. After homogenization and centrifugation at 3000 rpm for 1 min, heptane is eliminated. This step is repeated 5 times, and then the NaH is dried under Nitrogen. Next, 200 $\mu$L of anhydrous dimethyl sulfoxide, 100 $\mu$L of test solution (2-3 mg of product in 1 mL of anhydrous dimethyl sulfoxide after a few minutes at 70 °C if necessary), and 100 $\mu$L of methyl iodide are added. After agitation for 10 minutes, the reaction is stopped in an ice bath. After 2 minutes, 2 mL of chloroform and 2 mL of water are added. After homogenization and centrifugation at 3000 rpm for 1 min, the aqueous phase is eliminated, and the chloroform phase is washed 5 times with water and then evaporated. The dry residue is dissolved in acetone (25 $\mu$L), and 0,5 mL of aqueous trifluoroacetic acid is added, then stored in a screw-cap tube at 100 °C for 4 hours, and completely evaporated under Nitrogen. The residue is dissolved in 1 mL of methanol and evaporated under nitrogen. This step is repeated 3 times. The residue is dissolved in 500 $\mu$L of sodium borohydride solution (20 mg/mL), and the solution is placed under gentle agitation at room temperature for two hours. The solution is acidified with an acetic acid/methanol mixture (1:9; 2 drops), and then completely evaporated under Nitrogen. Boric acid is evaporated by co-distillation with methanol (1 to 2 mL) (5 times). The dry residue is treated with acetic anhydride (0,5 mL) at 100 °C for 4 hours, then completely evaporated under Nitrogen. Next, 2 mL of chloroform and 2 mL of water are added. After homogenization and centrifugation at 3000 rpm for 1 min, the aqueous phase is eliminated and the chloroform phase is washed 6 times with water, and then evaporated under Nitrogen. The residue is dissolved in 200 $\mu$L to 400 $\mu$L of chloroform, then analyzed by gas chromatography (GC) and gas chromatography coupled with mass spectrometry (GC-MS). The gas chromatography is performed for example on a Scion 456 system with a flame ionization detector, using helium as carrier gas. The gas chromatography coupled with mass spectrometry is performed for example on a Scion 456-SQ system, using helium as carrier gas. A capillary column (length 30 m, inner diameter 0,32 mm, film thickness 0,25 $\mu$m) made of 100 % Dimethylpolysiloxane fused silica, for example, Agilent DB-1 ref 123-1032, may be used used. The temperature is programmed as follows: 0 minute at 120 °C, -> 230 °C at 2 °C per minute, 230 °C for 1 minutes, -> 300 °C at 10 °C per minute, 300 °C for 2 minutes. The flow is at 1.7 mL/min in constant flow.

Such hydroxypropyl-cyclodextrin is commercially available. For example, mention can be made of KLEPTOSE® HPB (Roquette).

Preferably, the substituted cyclodextrin according to the disclosure complies with the US monograph as in force on September 1st, 2023. Preferably, the substituted cyclodextrin according to the disclosure complies with the European monograph as in force on September 1st, 2023.

Preferably, the amount of substituted cyclodextrin (e.g., the amount of HPBCD), as dry weight, in the tablet core according to the disclosure, is equal to or higher than 5%, preferably equal to or higher than 10%, preferably equal to or higher than 20%, preferably equal to or higher than 30%, preferably equal to or higher than 35%; said percentage being expressed with respect to the total dry weight of said tablet core. It is preferably equal to or lower than 70%, preferably equal to or lower than 60%, preferably equal to or lower than 50%, preferably equal to or lower than 45%. It is for example equal to 40%.

The tablet core according to the disclosure typically includes ingredients other than the therapeutic protein and substituted cyclodextrin as described in the instant descriptions and claims, as long as it does not interfere with the desired properties of the tablet, notably in terms of safety and performance. Such additional ingredient may be included in the therapeutic protein powder, or they may be added separately, by blending them with the therapeutic protein powder. Example of such other ingredients are: additional active ingredients; fillers and/or binders e.g., sugars, sugar alcohols, hydroxypropyl methylcellulose (HPMC), microcrystalline cellulose (MCC), partially pregelatinized starch; buffers e.g., histidine / histidine HCl buffer; colors; flavors; lubricants, e.g. magnesium stearate or stearyl fumarate; protease inhibitors.

Preferably, the tablet core according to the disclosure includes a filler (sometime also referred to as "filler-binder" in the field of tableting), preferably a direct compression excipient, preferably selected from sugars, sugar alcohols, HPMC, MCC, partially pregelatinized starch, or from any mixture thereof. It is preferably MCC and/or partially pregelatinized starch, more preferably MCC. Preferably, the amount of filler (e.g., MCC) of the tablet core, as dry weight, is equal to or higher than 5%, preferably equal to or higher than 10%, preferably equal to or higher than 20%, preferably equal to or higher than 30%, preferably equal to or higher than 40%, preferably equal to or higher than 45%; said percentage being expressed with respect to the total dry weight of said tablet core. It is preferably equal to or lower than 80%, preferably equal to or lower than 70%, preferably equal to or lower than 60%, preferably equal to or lower than 55%. It is for example equal to 50%. Preferably, said filler is added separately from the therapeutic protein powder i.e., by blending said therapeutic protein powder with said filler.

Preferably, the tablet core according to the disclosure includes a buffer. Preferably, such buffer has a pH selected from 3 to 10, preferably from 4 to 9, preferably from 5 to 7, preferably from 5 to 6, preferably of 5.5. Preferably, the buffer according to the disclosure is Histidine / Histidine HCl. Preferably, the amount of buffer, as dry weight, in the tablet core, is equal to or higher than 0.1%, preferably equal to or higher than 0.5%, preferably equal to or higher than 1.0%, preferably equal to or higher than 1.5%, preferably equal to or higher than 2.0%; said percentage being expressed with respect to the total dry weight of said tablet core. It is preferably equal to or lower than 5.0%, more preferably equal to or lower than 4.0%, more preferably equal to or lower than 3.0%. It is for example equal to 2.4%. Preferably, said buffer is included in the therapeutic protein powder. In other words, preferably, the tablet core according to the disclosure comprises a powder obtained by drying a liquid comprising a therapeutic protein, a substituted cyclodextrin, and a buffer, preferably histidine buffer.

Tableting classically requires lubrication. It may be external or internal lubrication. In case of internal lubrication, the tablet core according to the disclosure includes a lubricant, preferably magnesium stearate, stearyl fumarate, or a mixture thereof. In this case, the amount of lubricant, as dry weight, in the tablet core, is preferably equal to or higher than 1.0%, said percentage being expressed with respect to the total dry weight of said tablet core. It is typically equal to or lower than 3.0%.

Preferably, the tablet core according to the disclosure does not contain protease inhibitors other than the substituted cyclodextrin according to the disclosure. Indeed, as shown in the example section I, the inventors have shown that such additional protease inhibitors are not needed when using the substituted cyclodextrin according to the disclosure.

In a preferred embodiment, the tablet core according to the disclosure consists of:

- 5 to 70% of a substituted cyclodextrin, preferably comprising or consisting of HPBCD;
- 1 to 30% of a therapeutic protein, preferably comprising or consisting of adalimumab;
- 5 to 80% of a filler;
- 0.1 to 5.0% of a buffer;
- optionally up to 3% of a lubricant
- optionally up to 5% of other ingredients;

said percentages being expressed as dry weight, with respect to the total dry weight of the tablet core and their sum being equal to 100%.

Preferably, the nature and amount of ingredients are as described in the claims and embodiments included in the present specification.

For example, the tablet core according to the disclosure may consists of:

- 5 to 70% of HPBCD;
- 1 to 30% of adalimumab;
- 5 to 80% of a filler, preferably comprising or consisting of MCC;
- 0.1 to 5.0% of a buffer, preferably comprising or consisting of a histidine / histidine HCl buffer;
- optionally up to 3% of a lubricant
- optionally up to 5% of other ingredients;

said percentages being expressed as dry weight, with respect to the total dry weight of the tablet core and their sum being equal to 100%.

The tablet core may be prepared by granulation (preferably dry granulation) and then by tableting the resulting granules, eventually after the addition of other ingredients (e.g., a lubricant). It may also be prepared by direct compression. Preferably, the tablet core according to the disclosure is obtained by direct compression. That is to say that it is obtained by blending the therapeutic protein powder and eventual additional ingredient(s), and then tableting the resulting blend, without performing a prior step of granulation.

In a preferred embodiment, the tablet core is obtained by:

(i) preparing a therapeutic protein powder by drying a liquid comprising a substituted cyclodextrin, a therapeutic protein, and eventual additional ingredient(s) (typically a buffer),
(ii) blending the therapeutic protein powder obtained in step (i) with additional ingredient(s) (typically a filler, which is preferably a direct compression excipient), and then,
(iii) tableting the resulting blend, without performing a prior step of granulation.

The tablet according to the disclosure is coated with a film-coating coating composition comprising a water-insoluble polymer and soluble fiber.

The term "water insoluble polymer" classically refers to polymers that do not completely dissolve in water, such as for example ethylcellulose, certain starch derivatives or acrylic acid/meth-acrylic acid derivatives. In particular, it classically refers to polymers that do not completely dissolve in water at room temperature (i.e., 18 to 22 °C)

Preferably, the water-insoluble polymer according to the disclosure is selected from ethylcellulose, cellulose derivatives, acrylic and/or methacrylic ester polymers, polymers or copolymers of acrylate or methacrylate polyvinyl esters, starch derivatives, polyvinyl acetates, polyacrylic acid esters, butadiene styrene copolymers methacrylate ester copolymers, cellulose acetate phtalate, polyvinyl acetate phtalate, shellac, methacrylic acid copolymers, cellulose acetate trimellitate, hydroxypropyl methylcellulose phtalate, zein, starch acetate, or from any mixture thereof. It is more preferably ethylcellulose.

Preferably, the amount of water insoluble polymer, as dry weight, in the film-coating composition according to the disclosure, is equal to or higher than 30%, preferably equal to or higher than 40%, preferably equal to or higher than 50%; said percentage being expressed with respect to the total dry weight of said film-coating composition. It is preferably equal to or lower than 80%, preferably equal to or lower than 70%. It is for example selected from 50 to 70% or from 50 to 65%.

The term "soluble fiber" classically refers to saccharides which are not or only partially digested in the intestine by the action of acids or digestive enzymes present in the human upper digestive tract (small intestine and stomach) but which are at least partially fermented by the human intestinal flora. The term "soluble" is understood to mean soluble in water at room temperature (i.e., from 18 to 22 °C).

Soluble fibers according to the disclosure may be selected from xylooligosaccharides, inulin, oligofructoses, fructo-oligosaccharides (FOS), lactulose, galactomannan, suitable hydrolysates thereof, indigestible polydextrose, indigestible dextrins, partial hydrolysates thereof, trans-galacto-oligosaccharides (GOS), xylo-oligosaccharides (XOS), acemannans, lentinans, beta-glucans, partial hydrolysates thereof, polysaccharides-K (PSK), indigestible maltodextrins, partial hydrolysates thereof, or from any mixture thereof.

Preferably, the soluble fiber according to the disclosure is a soluble indigestible maltodextrin, a soluble indigestible dextrin, or a mixture thereof. It is more preferably a soluble indigestible dextrin.

Preferably, the soluble indigestible maltodextrin according to the disclosure is a branched maltodextrin. Preferably, the soluble indigestible dextrin according to the disclosure is a branched dextrin.

Preferably, the soluble fiber according to the disclosure has from 15 to 35% of 1->6 glucoside linkages, preferably from 20 to 35%, preferably from 25 to 35%. This percentage of 1->6 glucoside linkages may be determined by the person skilled in the art by using the conventional methylation technique described in HAKOMORI, S., 1964, J. Biol. Chem., 55, 205.

Preferably, the soluble fiber according to the disclosure has a reducing sugar content, as dry weight, of less than 20%, preferably equal to or lower than 15%, preferably equal or lower than 10%; said percentage being expressed with respect to the total dry weight of soluble fibers. This reducing sugar content may be determined by the person skilled in the art according to the well-known "Bertrand method" by precipitation with cuprous oxide in reducing media, filtration on a sintered-glass filter, and weighting of the residue.

Preferably, the soluble fiber according to the disclosure has a number-average molecular weight (Mn) equal to or lower than 10 000 g/mol, preferably equal to or lower than 8 000 g/mol, preferably equal to or lower than 7 000 g/mol, preferably equal to or lower than 6 000 g/mol, preferably equal to or lower than 5 000 g/mol, preferably equal to or lower than 4 500 g/mol. Preferably, this Mn is equal to or higher than 100 g/mol, preferably equal to or higher than 200 g/mol, preferably equal to or higher than 300 g/mol.

Preferably, the soluble fiber according to the disclosure has a weight-average molecular weight (Mw) equal to or lower than 20 000 g/mol, preferably equal to or lower than 10 000 g/mol, preferably equal to or lower than 9 000 g/mol, preferably equal to or lower than 8 000 g/mol, preferably equal to or lower than 7 000 g/mol, preferably equal to or lower than 6 000 g/mol. Preferably, this Mw is equal to or higher than 100 g/mol, preferably equal to or higher than 500 g/mol, preferably equal to or higher than 700 g/mol.

Preferably, the soluble fiber according to the disclosure has a polymolecularity index (Mw/Mn) lower than 5, preferably lower than 4, preferably lower than 3. It is in general equal to or higher than 1, even equal to or higher than 2. It is for example from 1 to 3, or from 1.0 to 3.0.

The Mn and Mw may be determined by the person skilled in the art by gel-permeation chromatography (GPC) on calibrated chromatographic columns using pullulans standards.

Preferably, the fiber according to the disclosure is derived from wheat starch.

Example of suitable soluble fiber according to the disclosure is commercially available. Mentioned can be made for example of NUTRIOSE® FB 06 (Roquette).

Preferably, the amount of soluble fiber, as dry weight, in the film-coating composition according to the disclosure, is equal to or higher than 5%, preferably equal to or higher than 10%, preferably equal to or higher than 12%, preferably equal to or higher than 13%, preferably equal to or higher than 14%; said percentage being expressed with respect to the total dry weight of said film-coating composition. It is preferably equal to or lower than 40%, preferably equal to or lower than 35%, preferably equal to or lower than 30%, preferably equal to or lower than 25%. It is for example selected from 14 to 25%.

Preferably, the water-insoluble polymer:soluble fiber dry weight ratio in the film-coating composition according to the disclosure is selected from (1:1) to (8:1), preferably from (1:1) to

(7:1), preferably from (1:1) to (6:1), preferably from (1:1) to (5:1), preferably from (1.0:1.0) to (5.0:1.0), preferably from (1.5:1.0) to (4.5:1.0), preferably from (2.0:1.0) to (4.0:1.0), preferably from (2.2:1.0) to (4.0:1.0), preferably from (2.3:1.0) to (4.0:1.0), preferably from (2.4:1.0) to (3.9:1.0).

The film-coating composition according to the disclosure may include ingredients other than the one mentioned before, as long as it does not interfere with the desired properties, notably in term of safety and properties of the film-coating composition obtained therefrom. Such other ingredient may for example be selected from:

- pigments and opacifiers (e.g., titanium dioxide, calcium carbonate, lakes);
- flavors, sweeteners;
- plasticizers;
- surfactants and/or emulsifiers, for example sodium lauryl sulphate, cetyl alcohol;

- active ingredients, for example pharmaceutical, nutraceutical, or veterinary active ingredients;
- or from any mixture thereof.

Preferably, the film-coating composition according to the disclosure comprises a plasticizer, preferably selected from triethyl citrate (TEC), polyethylene glycol (PEG), glycerin, triacetin, sodium lauryl sulfate, diethyl phthalate (DEP), dibutyl phthalate (DBP), dibutyl sebacate, or from any mixture thereof. It is preferably triethyl citrate.

Preferably, the amount of plasticizer, as dry weight, in the film-coating composition according to the disclosure, is equal to or higher than 5%, preferably equal to or higher than 10%, preferably equal to or higher than 12%, preferably equal to or higher than 13%, preferably equal to or higher than 14%; said percentage being expressed with respect to the total dry weight of said film-coating composition. It is preferably equal to or lower than 40%, preferably equal to or lower than 35%, preferably equal to or lower than 30%, preferably equal to or lower than 25%, preferably equal to or lower than 20%. It is for example selected from 14 to 20%.

Preferably, the amount of surfactants and/or emulsifiers, as dry weight, in the film-coating composition according to the disclosure, is equal to or higher than 0%, preferably equal to or higher than 1%, preferably equal to or higher than 2%, preferably equal to or higher than 3%, preferably equal to or higher than 4%, preferably equal to or higher than 5%; said percentage being expressed with respect to the total dry weight of said film-coating composition. It is preferably equal to or lower than 15%, preferably equal to or lower than 14%, preferably equal to or lower than 13%, preferably equal to or lower than 12%, preferably equal to or lower than 11%. It is for example selected from 5 to 11%.

These surfactants and emulsifiers are typically coming from the introduction of the water-insoluble polymer in the film-coating composition, when said water-insoluble polymer is introduced in the form of a dispersion or of a suspension. Indeed, preferably, the water-insoluble polymer according to the disclosure is introduced into the film-coating formulation into the form of an aqueous dispersion comprising said water-insoluble polymer, and at least one of a surfactant or an emulsifier. This way, the used of organic solvents can be avoided. Indeed, although the water-insoluble polymer is, by definition, not soluble in water, organic-solvent based coating has been being phased out by industry due to environmental and safety concerns. Therefore, coating with aqueous formulations is preferred in industry. This is why, preferably, the water-insoluble polymer according to the disclosure is formulated as a latex dispersion or suspension (preferably nanosuspension) comprising surfactant(s) and/or emulsifier(s).

Examples of such suspensions are commercially available. Mention can be made for example of AQUACOAT® ECD (IFF), an aqueous ethylcellulose dispersion comprising sodium lauryl sulphate and cetyl alcohol, SURELEASE® (Colorcon), an aqueous ethylcellulose dispersion stabilized by ammonium solution.

Preferably, the amount of titanium dioxide, as dry weight, in the film-coating composition according to the disclosure, is equal to or lower than 5%, preferably equal to or lower than 4%, preferably equal to or lower than 3%, preferably equal to or lower than 2%, preferably equal to or lower than 1%, preferably equal to or lower than 0.5%, preferably equal to or lower than 0.10%, preferably equal to or lower than 0.005%; said percentage being expressed with respect to the total dry weight of said film-coating composition. More preferably, the film-coating composition according to the disclosure does not include titanium dioxide.

For the film-coating composition to be used, it is typically in the liquid state. The solvent used may be an organic solvent or water.

Therefore, in a preferred embodiment, the film-coating composition according to the disclosure is a liquid composition comprising a solvent, preferably a polar solvent, preferably water. In other words, it is preferably an aqueous film-coating composition.

In general, the liquid coating composition according to the disclosure has a dry matters content equal to or higher than 10%, preferably equal to or higher than 15%, preferably equal to or higher than 20%; said percentage being expressed as dry weight with respect to the total weight of said film-coating composition. In general, the dry weight of the liquid film-coating composition is equal to or lower than 70%, preferably equal to or lower than 65%, preferably equal to or lower than 60%, preferably equal to or lower than 55%, preferably equal to or lower than 50%, preferably equal to or lower than 40%, preferably equal to or lower than 30%. It is for example selected from 20 to 30%.

Preferably, the water-insoluble polymer according to the disclosure is included in the film-coating composition according to the disclosure in the form of an aqueous suspensions. Therefore, in a preferred embodiment, the film-coating composition according to the disclosure is obtained or obtainable by blending a soluble fiber, a suspension of water-insoluble polymer, and optionally, other ingredients.

In another preferred embodiment, the film-coating composition according to the disclosure is a ready-to-use powdery composition. In that case, the film-coating composition is mixed with a solvent, preferably water, before use.

Alternatively, although not preferred, coating can be performed by dry coating, for example by using a powdery blend comprising the soluble fiber and the water-insoluble polymer (e.g., micronized ethylcellulose).

Preferably, the film-coating composition according to the disclosure consists of:

(a) dry matters, said dry matters consisting of:

- (a.1) a water-insoluble polymer;
- (a.2) a soluble fiber;
- (a.3) a plasticizer;
- (a.4) optionally a surfactant and/or an emulsifier;
- (a.5) optionally other ingredients;

(b) optionally a solvent, preferably water.

Preferably, said film-coating composition is as described in the claims and embodiments included in the present specification. Preferably, the water-insoluble polymer is as described in the claims and embodiments included in the present specification. Preferably, the soluble fiber is as described in the claims and embodiments included in the present specification. Preferably, the plasticizer is as described in the claims and embodiments included in the present specification. Preferably, the surfactant and/or an emulsifier is as described in the claims and embodiments included in the present specification. Preferably, the amounts of ingredients are as described in the claims and embodiments included in the present specification.

The coating level of the tablet according to the disclosure typically depends on the tablet shape and size, and may be adapted accordingly. Preferably, the film-coated tablet according to the disclosure is coated with the film-coating composition according to the disclosure at a percentage of coating (%coating) higher than 1%, preferably equal to or higher than 5%, preferably equal to or higher than 10%, preferably equal to or higher than 15%, preferably equal to or higher than 20%, preferably equal to or higher than 21%, preferably equal to or higher than 22%, preferably equal to or higher than 23%, preferably equal to or higher than 24%, preferably equal to or higher than 25%. It is preferably equal to or lower than 50%, preferably equal to or lower than 45%, preferably equal to or lower than 40%, preferably equal to or lower than 35%. It is for example from 20 to 35% or from 25 to 30%.

This %coating, also referred to as "mass gain" or "coating level", is determined as follows:

[Math. 1]

$$\% \, coating = \frac{weight \, of \, tablets \, after \, coating \, - \, weight \, of \, tablets \, before \, coating}{weight \, of \, tablets \, before \, coating} \times 100$$

The tablet core according to the disclosure may be coated with one or more coating composition(s) other than the film-coating composition according to the disclosure, as long as it does not interfere with the desired properties of the tablet, notably in terms of safety and performance. Such other coating may be applied before or after the film-coating according to the disclosure.

**Process for Making the Tablets**

The present invention is also directed to a process for preparing a colon targeted tablet according to the disclosure comprising:

a step (a) of obtaining a tablet core according to the disclosure i.e., comprising a therapeutic protein powder, said protein powder being obtained by drying a liquid comprising a therapeutic protein and a substituted cyclodextrin; and, a step (b) of coating said tablet core with a film-coating composition according to the disclosure, preferably to a %coating higher than 15%.

Preferably, the therapeutic protein powder is as described in the claims and embodiments included in the present specification. Preferably, the therapeutic protein is as described in the claims and embodiments included in the present specification. Preferably, the cyclodextrin is as described in the claims and embodiments included in the present specification. Preferably, the tablet core is as described in the claims and embodiments included in the present specification. Preferably, the film-coating composition is as described in the claims and embodiments included in the present specification. Preferably the soluble fiber is as described in the claims and embodiments included in the present specification. Preferably, the water-insoluble polymer is as described in the claims and embodiments included in the present specification.

Step (a) may be performed by granulation and then tableting (preferably dry granulation), or by direct compression. Preferably, step (a) is performed by direct compression. In other words, it is performed by blending the therapeutic protein powder and eventual additional ingredients, and then tableting said blend, without any prior step of granulation.

Preferably, the step (a) is performed by:

(a.i) preparing a therapeutic protein powder according to the disclosure, in particular by drying a liquid comprising a substituted cyclodextrin, a therapeutic protein, and optionally a buffer, then,

(a.ii) blending the powder obtained in step (a.i) with additional ingredient(s) (typically a filler, which is preferably a direct compression excipient), and then,

(a.iii) tableting the resulting blend so as to obtain a tablet core.

Step (a.i) may be performed by freeze-drying. It may also be performed for example by spray-drying, spray-freeze drying, electrospray, electrospray drying, vacuum drying, or thin film freeze drying.

In an embodiment, the process for preparing a colon targeted tablet according to the disclosure comprises at least one additional step of coating (film-coating and/or sugar coating), performed before or after step (b).

Typically, the step (b) of coating is a step of dispersing or dissolving the water-insoluble polymer, the soluble fiber and optional other ingredients into a suitable solvent, followed by a step of spraying the liquid thus obtained onto a bed of tablet cores in motion.

In an embodiment, the water-insoluble polymer is included in the film-coating composition according to the disclosure in the form of an aqueous suspension or dispersion, preferably as described in the claims and embodiments included in the present specification. Therefore, in a preferred embodiment, the film-coating composition is obtained by blending a soluble fiber, a suspension or dispersion of a water-insoluble polymer, and optionally, other ingredients (in particular a plasticizer).

Preferably, the step (b) is followed by a step (c) of curing the film-coating.

Preferably, this curing step (c) is performed at a temperature higher than 25 °C, preferably equal to or higher than 30 °C, preferably equal to or higher than 35 °C. This curing temperature is preferably lower than 60 °C, preferably equal to or lower than 55 °C, preferably equal to or lower than 50 °C, preferably equal to or lower than 45 °C. it is for example from 35 to 45 °C, or equal to 40 °C.

Preferably, this curing step (c) is performed at a relative humidity (RH) equal to or higher than 40%, preferably equal to or higher than 60%, preferably higher than 60%, preferably equal to or higher than 65%, preferably equal to or higher than 70%. It is preferably equal to or lower than 90%, preferably equal to or lower than 85%, preferably equal to or lower than 80%. It is for example from 70 to 80%, or equal to 75%.

Preferably, the duration of this curing step (c) is higher than 15 hours, preferably equal to or higher than 16 hours, preferably equal to or higher than 17 hours, preferably equal to or higher than 18 hours. It is preferably lower than 36 hours, preferably equal to or lower than 48 hours, preferably equal to or lower than 24 hours. It is for example from 16 to 48 hours, or from 18 to 48 hours, or from 18 to 24 hours.

The instant disclosure also relates to tablets obtainable by or obtained by the process according to the disclosure.

## Use as a Medicament

The present invention also relates the use of the colon targeted tablet according to the disclosure as a medicament.

Preferably, the use is for the treatment of a disease selected from inflammatory bowel diseases (IBDs), preferably selected from Crohn disease, and ulcerative colitis.

Preferably, in particular if the tablet is for the treatment of a human, the quantity of therapeutic protein administered is higher than 5 mg/kg per day, preferably equal to or higher than 10 mg/kg per day, or equal to or higher than 15 mg/kg per day. it is preferably equal to or lower than 100 mg/kg per day, preferably equal to or lower than 90 mg/kg per day, preferably equal to or lower than 80 mg/kg per day, preferably equal to or lower than 70 mg/kg per day, preferably equal to or lower than 60 mg/kg per day, preferably equal to or lower than 50 mg/kg per day, preferably equal to or lower than 40 mg/kg per day, preferably equal to or lower than 30 mg/kg per day, preferably equal to or lower than 20 mg/kg per day, even lower than 15 mg/kg..

It is reminded that the therapeutic protein is preferably an antibody, more preferably adalimumab.

The present invention also relates to a method for treating an organism in need thereof comprising administering orally to said organism, the colon targeted tablet according to the disclosure.

Preferably, the organism to be treated is a mammal, preferably a human.

Preferably, the organism to be treated is suffering from an inflammatory bowel disease (IBD), preferably selected from Crohn disease and ulcerative colitis.

## Remarks

The amounts of ingredients may be expressed in percentages by weight. These weights are amounts of ingredients as such, in their typical powdery or oily form (or liquid form for solvents). All compounds may include small amounts of impurities. Powdery ingredients may also include small amount of water (also referred to as %moisture or as "loss on drying").

However, if the amounts are expressed as "dry weights", this refers to amounts of anhydrous ingredients i.e., without the water. Similarly, "dry matters" refer to anhydrous matters.

Where an amount of ingredient in a product is selected in a range of from 0 to X% or expressed as being "less than X%" or "lower than X%", it is understood that an embodiment where said product is free of said ingredient is included.

Also, when referring to amounts of ingredients in products such as the tablet core or the coating composition, it is classically understood that these amounts are those of the blend used for making said product. These amounts might differ (to a small extent) from the amounts actually found in the final product (that may actually not be measurable), for example due to small material losses or due to a small heterogeneity of the blend used for making the product.

Also, where a material is defined in generic terms (e.g., substituted cyclodextrin, filler...), in embodiments where the latter are selected among a list (from 1 to n species), said list can be used interchangeably with said generic term.

- For example, where the disclosure reads "in a preferred embodiment, the substituted cyclodextrin according to the disclosure is HPBCD", it is considered that the following disclosure "Preferably, the amount of substituted cyclodextrin in the tablet is equal to or higher than 5%" also discloses the following embodiment: "Preferably, the amount of HPBCD in the tablet is equal to or higher than 5%". This embodiment does not exclude the presence of other substituted cyclodextrin(s) in different amounts.
- As another example, where the specification reads "Preferably, the tablet core according to the disclosure includes a filler, [...] preferably selected from sugars, sugar alcohols, HPMC, MCC, partially pregelatinized starch or from any mixture thereof.", it is considered that the following disclosure "Preferably, the amount of filler of the tablet is equal to or higher than 5%" also discloses the following embodiment: "Preferably, the tablet includes a compound selected from sugars, sugar alcohols, HPMC, MCC, partially pregelatinized starch or any mixture thereof in an amount that is equal to or higher than 5%".

Still where a material is selected among a list, it is considered that embodiments where said material comprises or consist of said list are also disclosed. For example, where the disclosure reads "in a preferred embodiment, the substituted cyclodextrin according to the disclosure is HPBCD", the followings:

- "in a preferred embodiment, the substituted cyclodextrin according to the disclosure consists of HPBCD" and
- "in a preferred embodiment, the substituted cyclodextrin according to the disclosure comprises HPBCD"

are also considered to be disclosed.

It is understood that in the first case, HPBCD is the sole substituted cyclodextrin of the embodiment, while in the second case, other substituted cyclodextrins may be present.

Where a product or composition comprises "other ingredient(s)" it classically means any ingredient that doesn't meet the definition of the ingredients otherwise listed in the considered embodiment. In other words, where a generic composition Y comprises a compound A which can be selected from A', A", or from a mixture thereof: in the embodiment where the generic term Y is substituted by the species A', compound A" is considered to be an "other ingredient".

It is reminded that, as used in the specification and the appended claims, the singular form "a," "an," and "the" comprise plural referents unless the context clearly indicates otherwise, and means "at least one" or "one or more". For example, reference to a component in the singular is intended to comprise a plurality of components. Thus, for example, "a" therapeutic protein or "a" substituted cyclodextrin, means "one or more" therapeutic protein or "one or more" substituted cyclodextrin, respectively.

Other characteristics and advantages of the present invention will emerge clearly on reading the examples given hereinafter, which illustrate the invention without however limiting it.

**Examples**

**A- Determination of film curing conditions and %coating for modified release**

**A-I Introduction**

Oral bioavailability of therapeutic proteins is low due to physical and enzymatic degradation during gastrointestinal passage. The highly acidic pH of the gastric environment results in protein unfolding and ensuing hydrolysis by protease enzymes in the stomach and small intestines.

To overcome this bioavailability challenge, the inventors proposed to deliver the protein directly to its target site in the colon with colon-targeting coatings. To this end, the tablet was coated with a water-insoluble polymer (ethylcellulose) and a soluble fiber (NUTRIOSE® FB 06). The water-insoluble polymer hinders premature film dissolution and drug release in the upper gastrointestinal tract. Upon reaching the colon, the soluble fiber is fermented by bacteria in the colon, resulting in disintegration of the film coating and subsequent drug release.

Ethylcellulose based film coatings require a thermal treatment (curing step) to promote particle coalescence for complete film formation. Ethylcellulose films plasticized with triethyl citrate are conventionally cured at an elevated temperature of 60 °C for up to 24 h. Nevertheless, exposure of the labile protein macromolecule to high temperatures is detrimental to its structure and activity.

In this study, we set out to investigate alternative, unconventional curing conditions which are more favorable towards preserving protein stability, at the same time achieving complete film coalescence for delayed drug release.

## A-II Material and Methods

Tablets containing theophylline (wt. 10%), microcrystalline cellulose (MICROCEL® 302, Roquette) (89.5 wt.%) and magnesium stearate (0.5 wt.%) were compacted with a 5 mm round, concave, Euro-B tablet punch and die set using Piccola rotary tablet press (Riva). and coated to 40% coating, with a film-coating composition comprising ethylcellulose and a soluble fiber, plasticized with triethylcitrate (TEC) (25% TEC, expressed as dry weight with respect to the dry weight of ethylcellulose (by assuming that the ethylcellulose dispersion used had 30% (w/w), as dry weight, of ethylcellulose).

The following polymer materials were used for the film-coating:

- soluble fiber: NUTRIOSE® FB 06 (ROQUETTE, Lestrem, France);
- water-insoluble polymer: aqueous ethylcellulose dispersion AQUACOAT® ECD 30 (FMC Biopolymer, Philadelphia, USA), which consists in 24.5 to 29.5% of ethylcellulose, 0.9 to 1.7% of sodium lauryl sulfate and 1.7 to 3.3% cetyl alcohol (it was considered that said percentages, provided by the supplier, are expressed as dry weight with respect to the total weight of the dispersion).

Note that because the aqueous ethylcellulose dispersion (AQUACOAT® ECD 30) can include from 24.5 to 29.5% of ethylcellulose, for this experiment and in all foregoing experiments, for calculating the ratios of ethylcellulose to NUTRIOSE®, it was considered that AQUACOAT® ECD 30 had 30% ethylcellulose. In this experiment, the dry weight ratio ethylcellulose: NUTRIOSE® was of (4:1) (meaning that, in practice, it is somewhere from (3.3:1.0) to (3.9:1.0)).

The coated tablets were exposed to different curing conditions and duration as shown in Table 1. Results are also showed in Table 1.

Then, tablets were prepared at 15% and 30% coating. Sodium chloride (Merck, Denmark), hydrochloric acid (Merck, Germany), and purified water (Advantage A10, MilliQ, USA) were used to prepare United States Pharmacopoeia (USP) simulated gastric fluid (SGF), which was used as the dissolution media in the acid stage. In the buffer stage, the pH 1.2 SGF was converted to pH 6.8 simulated intestinal fluid (SIF) by adding 0.2 M tribasic sodium phosphate (Merck, Germany). The tablets were incubated 2 hours in SGF and 9 hours in SIF. At the end of dissolution, for determining the total drug content of the tablets, tablets were crushed, and spin down performed at 250 rpm. Samples were collected at 60 and 90 min. %drug release was calculated as follow: %drug release = Amount of drug released at each time point / Drug content per tablet) x 100.

Theophylline release was measure by HPLC, using the following parameters: Column: Kinetex $2.6\mu$m C18 100Å; Isocratic; Flow rate: 0.3 mL/min; Mobile Phase C: $H_2O$ with 0.1% trifluoroacetic acid; Autosampler temperature 25 °C; Detector conditions = 272 nm; Peakwidth > 0.013min (0.25s response time, 20Hz); Run time: 6 min; Back pressure during equilibration: 250 bar.

Results are showed in Figure 1.

## A-III Results and Discussion

[Table 1] Film disintegration time of coated tablets (40%coating) cured at different curing conditions (temperature, RH, and time).

| Curing conditions | 3h | 6h | 9h | 15h | 18h | 24h | 48h |
|---|---|---|---|---|---|---|---|
| 60 °C, about 60-65% RH | - | - | - | - | - | <4h | 5.4±0.6h |
| 40 °C, ambient RH | - | - | - | - | - | <1.5h | 1.7±0.2h |

(continued)

| Curing conditions | 3h | 6h | 9h | 15h | 18h | 24h | 48h |
|---|---|---|---|---|---|---|---|
| 40 °C, 75 % RH | 3.1 ±0.3h | 4.1 ±0.1h | 5.3 ±0.2h | 6.8 ±0.1h | >8h | >8h | >8h |
| 25 °C, 75 % RH | 0.8 ±0.0h | 0.7 ±0.1h | 0.8 ±0.0h | 0.7 ±0.1h | 0.8 ±0.1h | 0.7 ±0.1h | - |
| - : condition not tested | | | | | | | |

Mean gastric and small intestinal transit times in patients with IBD can be up to 2 hours and 6 hours, respectively. As such, the film coating should remain intact for a minimum of 8 hours to suppress premature drug release in the upper gastrointestinal tract before the colon is reached (Table 1).

Film coatings cured at 60 °C and ambient humidity up to 48 h was unable to maintain its integrity for the required duration. In addition, the elevated temperature may be detrimental to protein stability (Table 1).

Curing the coated tablets at a high humidity level of 75% and lower temperature of 40 °C for 24 h produced film coatings with better integrity which remained intact for more than 8 hours (Table 1).

Coating level of 30% suppressed drug release in simulated gastric dissolution media (2 h) and simulated small intestinal dissolution media (9 h). A coating level of 15% did not produce a delayed release profile. Film coating and tablet disintegration were observed within 30 min (Figure 1).

**B- Development work on human plasma immunoglobulin G (human plasma igG)**

**B-I Introduction**

The aim of this study was to stabilize the model protein, human plasma IgG via freeze drying with a stabilizer in order to preserve its structure and activity throughout oral solid dosage form manufacture. To this end, hydroxypropyl-β-cyclodextrin (HPBCD) was tested.

**B-II Material and Methods**

First, to demonstrate the detrimental effect of oral dosage form processing on human plasma IgG, a trial was performed in which IgG (BOC Sciences) was pulverized and blended at 5% (w/w) with 91.2% (w/w) microcrystalline cellulose (MICROCEL® 302, Roquette) and 3.8% (w/w) sodium starch glycolate, and subjected to tableting, coating simulation and curing.

Tableting was performed using compaction simulator (Styl'One, MedelPharm, France) equipped with 5 mm, round, concave tablet punch (Natoli), at a compression force of 0.8 - 1 kN to give tablets with tensile strength values in the range of 3 to 5 MPa. The tablets were subjected to heat stress simulating the coating process at 40 °C for 6 hours, followed by heat and humidity stress at 40 °C / 75% relative humidity for 24 hours to simulate film curing conditions.

Human plasma IgG stability was determined by size exclusion chromatography (SEC) as percentage of monomer recovery against IgG standard ("IgG Control"), and after each process step of tableting ("Tablet"), simulated tablet coating ("Coating Sim."), and simulated film curing ("Curing Sim."). Results are showed in Figure 2.a).

For testing HPBCD as a stabilizer, human plasma IgG (BOC Sciences) was first lyophilized with or without HPBCD (KLEPTOSE® HPB, Roquette). Human plasma IgG solutions were prepared at a concentration of 12.5 mg/mL in 25mM sodium phosphate buffer, pH 6.8 without HPBCD ("Buffer" samples), with HPBCD 12.5% w/v ("HPBCD 12.5%" samples), and with HPBCD 25% w/v ("HPBCD 25%" samples). The sample solutions were freeze-dried using an Epsilon 2-4 or 2-6 LSCPlus freeze dryer (Martin Christ) at a freezing temperature of -30 °C for 1 hour, followed by primary and secondary drying at -20 °C and 20 °C, respectively, at a vacuum pressure of 0.1 mbar.

Test tablets ("HPBCD 12.5%" and "HPBCD 25%" items) were prepared from HPBCD 12.5%" and "HPBCD 25%" samples as follows: the lyophilizate was pulverized, and subjected to the same process of tableting, coating simulation and film curing simulation as above.

Control tablets ("Buffer" item) were prepared from "Buffer" samples as follows: the lyophilizate was pulverized, blended with HPBCD (12.62% (w/w) lyophilizate, 87.38% (w/w) HPBCD), and subjected to the same process of tableting, coating simulation and film curing simulation as above.

Human plasma IgG stability was determined by size exclusion chromatography (SEC) as percentage of monomer recovery against IgG standard, before freeze-drying ("Solution") and after each process step of freeze drying ("FD"), tableting ("Tablet"), simulated tablet coating ("Coating Stim."), and simulated film curing ("Curing Sim."). Results are showed in Figure 2.b).

**B-III Results and Discussion**

As expected, human plasma IgG is susceptible to mechanical, thermal and interfacial stress-induced degradation during solid dosage manufacturing process steps of tableting, coating and curing. This results in extensive loss of the protein during manufacturing (Figure 2.a)).

Freeze-drying human plasma IgG with HPBCD in (1:10) and (1:20) weight ratios stabilized the protein and prevented its degradation throughout solid dosage manufacturing steps of tableting, coating and film curing. Complete recovery throughout oral solid dosage manufacture was only achieved for IgG formulations co-freeze dried with HPBCD. "Buffer only" items showed incomplete recovery (Figures 2.b)).

Development work using human plasma IgG as the model protein demonstrated that freeze-drying with HPBCD stabilized the protein and preserved its physical stability throughout solid dosage manufacture.

**C- Adalimumab formulation in citrate buffer**

**C-I Introduction**

Adalimumab is a human monoclonal IgG1 antibody that targets both soluble and receptor-bound TNF-$\alpha$, one of the main cytokines in IBD pathogenesis. The commercial formulation of adalimumab HUMIRA® is a solution for subcutaneous injection in a citrate-phosphate buffer.

Monoclonal antibodies (mAb) are highly susceptible to physical and chemical stresses, such as heat, pH changes, shear stress, dehydration, surface adsorption and organic solvents, to name a few. Oral solid dosage manufacturing is especially fraught with unfavorable process conditions for labile biologics. This has deleterious effects on the protein's chemical and physical stability, leading to aggregation and precipitation. To date, no oral solid dosage form of adalimumab has been commercialized.

The aim of this study was to stabilize adalimumab *via* freeze drying with stabilizing excipients including hydroxypropyl-$\beta$-cyclodextrin (HPBCD), in order to preserve its structure and activity throughout oral solid dosage form manufacture.

**C-II Material and Methods**

Adalimumab was produced in-house by fed-batch culture and purified by protein A, anion exchange and cation exchange chromatography. Buffer exchange of purified adalimumab was performed using the NGC Discover chromatography system (Bio-Rad) and HiPrep 26/10 desalting column (Cytiva). Purified adalimumab was eluted with 50 mM citrate buffer at pH 5.5 and concentrated to ~25 mg/mL (adalimumab Stock Solution). HPBCD (KLEPTOSE® HPB, Roquette) was dissolved in 50 mM citrate buffer, pH 5.5 and made up to volume with adalimumab in citrate buffer to a final concentration of 12.5 mg/mL adalimumab and 12.5 %w/v or 25 %w/v HPBCD. Adalimumab sample solutions were rocked gently on a rocking platform for 10 min to ensure homogeneity.

Adalimumab sample solutions were freeze-dried using an Epsilon 2-4 or 2-6 LSCPlus freeze dryer (Martin Christ). Solutions were frozen at -40 °C for 2 hours, followed by primary and secondary drying at -20 °C and 20 °C, respectively, at a vacuum pressure of 0.1 mbar.

The lyophilizates were pulverized by passing through a 500 $\mu$m sieve. 50 mg of powder containing adalimumab was weighed and compacted using a 5 mm, round, concave tablet punch (Natoli) with the Styl'One Evolution compaction simulator (Medelpharm) at 0.8 kN compression force under external lubrication with magnesium stearate.

A control ("Buffer") was included comprising freeze-dried adalimumab without HPBCD. The lyophilizate was pulverized, blended with HPBCD (19.54% (w/w) lyophilizate, 80.46% (w/w) HPBCD) and subjected to the same process of tableting as above.

Adalimumab tablets were stored at 40 °C for 6 h to simulate heat stress during the coating process, followed by storage at 40 °C, 75% relative humidity for 24 h to simulate the film curing process.

Adalimumab samples were analyzed before freeze-drying ("Solution") and after each process step of freeze drying ("FD"), tableting ("Tablet"), and simulated tablet coating ("Coating Stim."), using size-exclusion chromatography-high performance liquid chromatography (SEC-HPLC; Waters) with XBridge BEH SEC column (200Å, 3.5$\mu$m, 7.8 x 300mm) at a column temperature of 30 °C. 10 $\mu$L of each sample was injected and eluted isocratically with 25 mM sodium phosphate and 150 mM sodium chloride, pH 6.8 at a flow rate of 0.5 mL/min. Due to tablet deliquescence during simulated curing process, adalimumab samples were not analyzed after the simulated curing step. Adalimumab stability was determined as % monomer recovery against standard (adalimumab Stock Solution, refer above). Results are showed in Figure 3.

**C-III** <u>Results and Discussion</u>

Near complete recovery of adalimumab was achieved in HPBCD formulations, compared to 82% recovery in the buffer-only formulation, after solid dosage manufacturing steps of freeze drying, tableting and coating simulation (Figure 3).

However, tablet deliquescence was observed during the curing process, attributed to the hygroscopicity of citrate.

Freeze drying adalimumab with HPBCD stabilized the protein and preserved its physical stability during solid dosage manufacture of tableting and simulated coating. However, the presence of citrate in the buffer system caused deliquescence during the curing process.

An alternative buffer system was then evaluated for the formulation of adalimumab (Examples section D).

**D- Adalimumab formulation in histidine buffer**

**D-I** <u>Introduction</u>

The commercial formulation of adalimumab HUMIRA® is stabilized in an acidic citrate buffer system at pH 5.2. In the previous study, we prepared adalimumab tablets from adalimumab citrate-buffered solutions. Adalimumab tablets prepared from the HPBCD formulations demonstrated near complete recovery after tableting and coating simulation. However, tablet deliquescence was observed during the curing process when tablets were exposed to heat and humidity.

The aim of this study was to evaluate an alternative buffer system to citrate for the formulation of adalimumab. The buffer salts selected should not exhibit deliquescent properties to preserve tablet integrity during curing.

**D-II** <u>Material and Methods</u>

Adalimumab was produced in-house by fed-batch culture and purified by protein A, anion exchange and cation exchange chromatography. Buffer exchange of purified adalimumab was performed using the NGC Discover chromatography system (Bio-Rad) and HiPrep 26/10 desalting column (Cytiva). Purified adalimumab was eluted with 20 mM histidine buffer at pH 5.5 and concentrated to ~25 mg/mL (adalimumab Stock Solution). HPBCD (KLEPTOSE® HPB, Roquette) was dissolved in 20 mM histidine buffer, pH 5.5 and made up to volume with adalimumab in histidine buffer to a final concentration of 12.5 mg/mL adalimumab and 12.5 %w/v or 25 %w/v HPBCD. Adalimumab sample solutions were rocked gently on a rocking platform for 10 min to ensure homogeneity.

Adalimumab sample solutions were freeze-dried using an Epsilon 2-4 or 2-6 LSCPlus freeze dryer (Martin Christ). Solutions were frozen at -40 °C for 2 hours, followed by primary and secondary drying at -20 °C and 20 °C, respectively, at a vacuum pressure of 0.1 mbar.

The lyophilizates were pulverized by passing through a 500 $\mu$m sieve. 50 mg of powder containing adalimumab was weighed and compacted using a 5 mm, round, concave tablet punch (Natoli) with the Styl'One Evolution compaction simulator (Medelpharm) at 0.8 kN compression force under external lubrication with magnesium stearate.

A control ("Buffer") was included comprising freeze-dried adalimumab without HPBCD. The lyophilizate was pulverized, blended with HPBCD (13.25% (w/w) lyophilizate, 86.75% (w/w) HPBCD) and subjected to the same process of tableting as above.

Adalimumab tablets were stored at 40 °C for 6 h to simulate heat stress during the coating process, followed by storage at 40 °C, 75% relative humidity or at 60°C, ambient relative humidity (60-65%) for 24 h to simulate the film curing process.

Adalimumab samples were analyzed before freeze-drying ("Solution") and after each process step of freeze drying ("FD"), tableting ("Tablet"), simulated tablet coating ("Coating Sim."), and simulated film curing ("Curing Sim."), using size-exclusion chromatography-high performance liquid chromatography (SEC-HPLC; Waters) with XBridge BEH SEC column (200Å, 3.5$\mu$m, 7.8 x 300mm) at a column temperature of 30 °C. 10 $\mu$L of each sample was injected and eluted isocratically with 25 mM sodium phosphate and 150 mM sodium chloride, pH 6.8 at a flow rate of 0.5 mL/min. Adalimumab stability was determined as % monomer recovery against standard (adalimumab Stock Solution). Results are showed in Figure 4.

**D-III** <u>Results and discussion</u>

Up to 99% recovery was achieved for adalimumab with HPBCD formulation after oral solid dosage manufacture, including tableting, simulated coating and curing steps. In contrast, only 68% recovery was demonstrated for the buffer-only formulation (Figure 4). No tablet deliquescence was observed during curing.

Adalimumab formulation with HPBCD in histidine buffer system was able to stabilize the protein and preserve its physical stability during solid dosage manufacture of tableting, simulated coating and curing. No tablet deliquescence was observed, making histidine buffer a better choice, as compared to citrate buffer.

**E- Overcoming tablet brittleness with the addition of filler-binder**

**E-I** Introduction

Tablets with high brittleness are susceptible to defects because of their lower ability to accommodate stress during tablet production, coating, storage, transportation, and handling. This is shown as the propensity of these tablets to easy chipping, high friability, and hidden defects.

Tablet breakage can occur upon impact during the coating process if they are too brittle. One strategy to solve the issue is reformulation with the addition of a plastic binder. In addition, the spray rate can be increased at the beginning of the colon targeted coating, to quickly apply coating layer which acts as protection for the tablets.

Tablets prepared from freeze-dried HPBCD + therapeutic proteins formulations had sufficient tensile strength but were brittle. Tablet breakage was observed during the fluid-bed coating process. Other characteristics of brittleness including formation of fines during fracture, and large variability in tensile strength measurements were also observed.

The aim of this study was to reduce tablet brittleness with the addition of filler-binders, in order to obtain tablets robust enough to withstand impact of collision during fluid-bed coating.

**E-II** Material and Methods

HPBCD 12.5% and 25% w/v solutions were prepared by dissolving HPBCD (KLEPTOSE® HPB, Roquette) in 20 mM histidine buffer, pH 5.5. They were then freeze-dried using Epsilon 2-4 freeze dryer (Martin Christ). Solutions were frozen at -40 °C for 2 hours, followed by primary and secondary drying at -20 °C and 20 °C, respectively, at a vacuum pressure of 0.1 mbar.

The lyophilizates were pulverized by passing through a 500 $\mu$m sieve. The effect of filler-binder was investigated by addition of 50% w/w microcrystalline cellulose (MICROCEL® 302, Roquette) or partially pregelatinized starch (LYCATAB® C, Roquette) to the pulverized lyophilizate by blending.

50 mg of powder was weighed and compacted using a 5 mm, round, concave tablet punch (Natoli) with the Styl'One Evolution compaction simulator (Medelpharm) under external lubrication with magnesium stearate. Single compression or double compression (precompression, followed by main compression) cycles were employed.

Tablets prepared from freeze-dried HPBCD 12.5% w/v solution were compacted to tensile strength values of 4 - 5 MPa, while tablets prepared from freeze-dried HPBCD 25% w/v solution were compacted to tensile strength values of 3 - 5 MPa.

Reference tablets were prepared by tableting MCC ("Reference MCC tablets") or HPBCD ("Reference HPBCD tablets") according to the same process of tableting as above.

Tablet brittleness testing was performed using the Friabimat SA-400 (Copley Scientific). 12 tablets, each weighing 50 mg, were loaded into a glass container for the tests. Friability test was conducted at 240 oscillations/min for 2 min; and stress test was conducted at 400 oscillations/min for 2 min.

Finally, tablets were subjected to a trial Wurster coating run in a fluidized bed system (Midi-Glatt). The coating formulation used was the same as described in Examples section A-II, with a dry weight ratio Ethylcellulose:NUTRIOSE® of (4:1). The coating was performed during a time sufficient to verify whether tablet breaking would occur. Results are showed in Tables 2 and 3.

**E-III** Results and discussion

[Table 2] Friability and stress test results for tablet brittleness testing

|  | Tensile strength (MPa) | Friability (%) | Stress test |
|---|---|---|---|
| *Reference MCC tablets* Single compression | 2 | 0.7 | No breakage |
| *Reference HPBCD tablets* Sinqle compression | 2 | 0.7 | - |
| *FD HPBCD 12.5%_hist* | | | |

(continued)

| Formulation | Tensile strength (MPa) | Friability (%) | Stress test |
|---|---|---|---|
| Sinqle compression without filler | 4-5 | 0.9 | 4/12 broken tablets |
| Double compression without filler | 4-5 | 1.0 | 5/12 broken tablets |
| Double compression with 50% MCC | 4-5 | 0.3 | No breakage |
| Double compression with 50% LYCATAB® C | 4-5 | 0.03 | No breakage |
| FD HPBCD 25%_hist | | | |
| Formulation | Tensile strength (MPa) | Friability (%) | Stress test |
| Sinqle compression without filler | 3-4 | 0.9 | 4/12 broken tablets |
| Double compression without filler | 3-4 | - | 3/12 broken tablets |
| Double compression with 50% MCC | 3-5 | 0.4 | No breakage |
| Double compression with 50% LYCATAB® C | 3-5 | 0.6 | No breakage |

[Table 3] Wurster coating run in fluidized bed system

| FD HPBCD 12.5%_hist | | |
|---|---|---|
| | Tensile strength (MPa) | Fluidization + Coating Test |
| Sinqle compression without filler | 4-5 | Tablet chipping |
| Double compression without filler | 4-5 | Chipping + 1/12 broken tablets |
| Double compression with 50% MCC | 4-5 | No breakage |
| Double compression with 50% LYCATAB® C | 4-5 | No breakage |
| FD HPBCD 25%_hist | | |
| Formulation | Tensile strength (MPa) | Fluidization + Coating Test |
| Single compression without filler | 3-4 | Tablet chipping + 2/12 broken tablets |
| Double compression without filler | 3-4 | Chipping + 2/12 broken tablets |
| Double compression with 50% MCC | 3-5 | No breakage |
| Double compression with 50% LYCATAB® C | 3-5 | No breakage |

Blending with filler-binders such as MCC and partially pregelatinized starch reduced tablet brittleness, resulting in no tablet breakage during fluidization and coating (Tables 2 and 3).

**F- Stability of adalimumab tablet formulation with filler-binder**

**F-I Introduction**

Study as presented in section D investigated adalimumab freeze-dried with 12.5% and 25% w/v HPBCD solution (corresponding to adalimumab : HPBCD (1:10) and (1:20) weight ratios). Study as presented in section E further identified that addition of 50% w/w filler-binder was useful for tableting, thus further reducing the drug load. However, low drug load is unfavorable because it increases pill burden for patients.

Our previous investigations to date have employed heat stress to simulate the coating process of adalimumab tablets. Nevertheless, the mAb is exposed to other stresses besides heat stress during coating, for example mechanical stress and interfacial stress of the protein at the tablet-coating interface etc.

The aim of this study was to explore a higher drug load by freeze-drying adalimumab with 6.25% w/v HPBCD solution (corresponding to adalimumab : HPBCD (1:5) weight ratio).

The robustness of adalimumab tablets formulated with filler-binder was also evaluated in an actual fluidized bed coating run.

**F-II** <u>Material and Methods</u>

Adalimumab was produced in-house by fed-batch culture and purified by protein A, anion exchange and cation exchange chromatography. Buffer exchange of purified adalimumab was performed using the NGC Discover chromatography system (Bio-Rad) and HiPrep 26/10 desalting column (Cytiva). Purified adalimumab was eluted with 20 mM histidine buffer at pH 5.5 and concentrated to ~25 mg/mL (adalimumab Stock Solution). HPBCD (KLEPTOSE® HPB, Roquette) was dissolved in 20 mM histidine buffer, pH 5.5 and made up to volume with adalimumab in histidine buffer to a final concentration of 12.5 mg/mL adalimumab and 6.25 %w/v or 12.5 %w/v HPBCD. Adalimumab sample solutions were rocked gently on a rocking platform for 10 min to ensure homogeneity.

Adalimumab sample solutions were freeze-dried using Epsilon 2-4 LSCPlus freeze dryer (Martin Christ). Solutions were frozen at -40 °C for 2 hours, followed by primary and secondary drying at -20 °C and 20 °C, respectively, at a vacuum pressure of 0.1 mbar.

The lyophilizates were pulverized by passing through a 500 $\mu$m sieve. Microcrystalline cellulose (MICROCEL® 302, Roquette) or partially pregelatinized starch (LYCATAB® C, Roquette) was added by blending to 50 %w/w. 50 mg of the powder thus obtained was weighed and compacted using a 5 mm, round, concave tablet punch (Natoli) with the Styl'One Evolution compaction simulator (Medelpharm) at 1 kN compression force under external lubrication with magnesium stearate.

A control ("Buffer") was included comprising freeze-dried adalimumab without HPBCD. The lyophilizate was pulverized, blended with MCC and HPBCD (20.8% (w/w) lyophilizate, 39.6% (w/w) MCC, 39.6% (w/w) HPBCD) and subjected to the same process of tableting as above.

To prepare the coating dispersion, NUTRIOSE® FB 06 (soluble fiber) was dissolved in purified water (15% w/w), blended with plasticized ethylcellulose aqueous dispersion (AQUACOAT® ECD 30 diluted at 15 % dry weight ethylcellulose with respect to the total weight of the dispersion, and plasticized with 25 % TEC as dry weight with respect to the dry weight of ethylcellulose, overnight stirring) at a dry weight ratio Ethylcellulose:NUTRIOSE® of (4:1) and stirred for 6 hours prior to coating.

Adalimumab tablets were mixed with 150 g of dummy tablets (so as to have enough weight of tablets for the coating process) and coated in a fluidized bed system with Wurster insert (Midi-Glatt) until a weight gain of 30% w/w was achieved. The process parameters were as follows: inlet temperature = 40 °C, spray rate 1.3 mL/min, atomizing air pressure 1 bar, fluidizing air pressure 0.7 bar. After coating, the tablets were further dried for 10 min and subsequently cured for 24 h at 40 °C, 75% relative humidity.

Adalimumab samples were analyzed before freeze-drying ("Solution"), and after each process step of freeze drying ("FD"), tableting ("Tablets"), coating ("Coated") and film curing ("Cured"), using size-exclusion chromatography-high performance liquid chromatography (SEC-HPLC; Waters) with XBridge BEH SEC column (200Å, 3.5$\mu$m, 7.8 x 300mm) at a column temperature of 30 °C. 10 $\mu$L of each sample was injected and eluted isocratically with 25 mM sodium phosphate and 150 mM sodium chloride, pH 6.8 at a flow rate of 0.5 mL/min. Adalimumab stability was determined as % monomer recovery against standard (adalimumab Stock Solution). Results are showed in Figure 5.

The binding activity of adalimumab to its target, TNF-$\alpha$ was evaluated on the adalimumab stock solution ("Adalimumab stock Solution"), before freeze-drying ("Adalimumab + HPBCD Solution"), and after each process step of freeze drying with HPBCD ("FD"), blending with MCC or partially pregelatinized starch ("Blend with MCC", "Blend with Partially pregel starch"), and tableting ("Tablets with MCC", "Tablets with Partially pregel starch"), by enzyme-linked immunosorbent assay (ELISA) (Abcam, ab237641). Quantitative determination of adalimumab was performed per experimental protocol provided by the supplier. Results are showed in Figure 6.

Biological activity of adalimumab was assayed before freeze-drying ("Adalimumab + HPBCD Solution"), and after each process step of freeze drying ("FD"), blending with filler ("Blend with MCC"), and tableting ("Tablets with MCC"), coating ("Coated") and film curing ("Cured"), using cell-based assay (PathHunter® adalimumab Bioassay Kit (Eurofins DiscoverX). The bioassay was designed to measure IkB degradation as a result of TNF alpha-mediated activation of the NF-kB signaling pathway. The experiment protocol was provided by the supplier. Results are showed in Figure 7.

**F-III** <u>Results and Discussion</u>

Prototypes including MCC or partially pregelatinized starch (LYCATAB® C) as a filler achieved up to 87% recovery, compared to 41% recovery in the non-stabilized formulation (Figure 5). Lower mAb recovery values (84 - 87 %) compared to previous trials (90 - 99%) are attributed to the use of higher tablet compression forces.

Adalimumab tablet prototypes achieved up to 100% recovery as measured by SEC-HPLC, and up to 98% as measured by ELISA (Figure 6). Adalimumab tablet prototypes thus demonstrated physical stability (SEC-HPLC) and TNF-$\alpha$ binding capacity (ELISA) post-processing.

Adalimumab tablet prototypes demonstrated physical stability as determined by SEC monomer recovery, and biological activity determined by cell-based assay, after processing steps of freeze drying, tableting, tablet coating and curing (Figure 7).

Adalimumab tablets containing 8% drug load were produced, coated and cured. Adalimumab formulation demonstrated physical stability, TNF-binding capacity and bioactivity.

## G- In vitro drug release of coated human plasma IgG tablets

### G-I Introduction

The pathophysiological conditions in the colon of patients suffering from IBDs can be significantly different from those in healthy subjects. Therefore, it is important for coating performance and drug release tests to be performed in conditions simulating the disease state in IBD patients.

The coating composition, as well as the size and geometry of tablet cores are relevant to adjust suitable film coating levels for colon-targeting.

The aim of this study was to evaluate the performance of ethycellulose-soluble fiber blends as tablet film coatings to provide site-specific release to the colon.

### G-II Material and Methods

Tablets containing 50% (w/w) human plasma IgG freeze-dried with HPBCD and 50% (w/w) MCC (MICROCEL$^®$ 302, Roquette) were mixed with theophylline-loaded tablets (dummy tablets) (350 g) and then coated with different ratios and coating level [dry weight ratio Ethylcellulose:NUTRIOSE$^®$ (3:1) at 27% and 30% coating; dry weight ratio (4:1) at 25% and 27% coating].

For making the film-coating composition, NUTRIOSE$^®$ FB 06 was dissolved in purified water (15 % w/w), blended with plasticized aqueous ethylcellulose dispersion ((AQUACOAT$^®$ ECD 30 diluted at 15 % dry weight ethylcellulose with respect to the total weight of the dispersion, and plasticized with 25 % TEC as dry weight with respect to the dry weight of ethylcellulose, overnight stirring) at an Ethylcellulose:NUTRIOSE$^®$ dry weight ratio of (3:1) and (4:1) and stirred for 6 h prior to coating.

Tablets were coated in a pan coater equipped with a nozzle insert (Solidlab 1, Bosch, Syntegon, Schopfheim, Germany). Process parameters were as follows: inlet temperature = 62 $\pm$ 2 °C, product temperature = 39 $\pm$ 2 °C, spray rate = 2 g/min, atomization pressure = 1.0 bar (PIR 2.21: 1 bar; PIR 2.41: 500 mbar), inlet air volume = 20 m3/h, spray nozzle diameter = 0.5 mm. After coating, the tablets were further dried for 10 min and subsequently cured in an oven for 24 h at 40 °C. Drum coater speed was 25 rpm.

Drug release from the coated tablets was measured using an experimental setup to simulate conditions in the gastrointestinal tract (GIT). To simulate transit through the upper GIT, the tablets were exposed to 0.1 M HCl for 1 h and subsequently to phosphate buffer pH 6.8 (USP 30) for 5 h in an USP Apparatus 3 (Bio-Dis). After that, the tablets were transferred into 120 mL flasks filled with 100 mL culture medium inoculated with feces from IBD patients, or culture medium free of feces and bacteria for reasons of comparison. The samples were agitated (80 rpm; Stuart, Cole-Parmer; Villepinte, France) at 37 °C under anaerobic conditions (AnaeroGen 2.5 L; Thermo Scientific; Illkirch, France) (horizontal shaker).

Culture medium was prepared by dissolving 1.5 g beef extract, 3 g yeast extract, 5 g tryptone, 2.5 g NaCl and 0.3 g L-cysteine hydrochloride hydrate in 1 L distilled water (pH 7.0 $\pm$ 0.2) and subsequent sterilization in an autoclave. Feces of patients with Crohn's Disease or Ulcerative Colitis as well as feces of healthy subjects were diluted 1:200 with cysteinated Ringer solution; 2.5 mL of this suspension was diluted with culture medium to 100 mL.

At pre-determined time points, 2 mL samples were withdrawn, centrifuged at 13 000 rpm for 5 min, filtered (0.22 $\mu$m) and analyzed for human plasma IgG content using enzyme-linked immunosorbent assay (ELISA). IgG was detected UV-spectrophotometrically at $\lambda$=450 nm. Results are showed in Figure 8.

### G-III Results and Discussion

Human plasma IgG release in the media simulating the transit through the upper GIT was effectively suppressed at 25 and 27 % coating level for Ethylcellulose:NUTRIOSE$^®$ coating blend in a (4:1) ratio (Figure 8).

A significant increase in the release rate was observed once the tablets were exposed to culture medium inoculated with fecal samples from IBD patients (Figure 8). The sudden increase in drug permeability can be attributed to NUTRIOSE$^®$ acting as a substrate for enzymes secreted by microflora in patients suffering from Crohn's Disease and Ulcerative Colitis.

Human plasma IgG release in the media simulating the transit through the upper GIT was effectively suppressed at 27 and 30 % coating level for Ethylcellulose:NUTRIOSE$^®$ coating blend in a (3:1) ratio (Figure 8).

A significant increase in the release rate was observed once the tablets were exposed to culture medium inoculated with fecal samples from IBD patients. Lag time upon exposure to fecal samples was increased with increasing coating level as well as ethylcellulose content in the coating blends (Figure 8).

These coating blends and ratios were then evaluated on adalimumab tablets (Examples section H).

### H- In vitro drug release of coated adalimumab tablets

#### H-I Introduction

The aim of this study was to evaluate the performance of ethycellulose-NUTRIOSE® blends as tablet film coatings to provide site-specific release to the colon for adalimumab tablets.

#### H-II Material and Methods

Same material and protocols as presented in Examples section G-II were used, except that human plasma IgG antibody was replaced by adalimumab. Results are showed in Figure 9.

#### H-III Results and Discussion

Release of adalimumab was effectively suppressed in release media simulating the conditions in the upper GIT when coated with Ethylcellulose:NUTRIOSE® (4:1) dry weight ratio to a coating level of 25%. Drug release was observed as soon as the tablets were exposed to fecal samples of IBD patients simulating the colonic phase (Figure 9).

Release of adalimumab was effectively suppressed in release media simulating the conditions in the upper GIT when coated with Ethylcellulose:NUTRIOSE® (3:1) dry weight ratio to a coating level of 30%. Drug release was observed as soon as the tablets were exposed to fecal samples of IBD patients simulating the colonic phase (Figure 9).

Some premature drug release can be observed in the upper GIT phase for the (3:1) ratio at 30% coating level (Figure 9).

Appropriate colon-targeted film coatings based on ethylcellulose and NUTRIOSE® were thus identified for adalimumab tablets:

- Ethylcellulose:NUTRIOSE® (4:1) ratio, 25% coating;
- Ethylcellulose:NUTRIOSE® (3:1) ratio, 30% coating.

Of the two, option (i) is preferred because there was no premature release observed in the upper GIT phase, compared to option (ii) whereby a small degree of drug release was observed.

### I- Stability of adalimumab in simulated colonic environment

#### I-I Introduction

Although the colon has conventionally been identified to have reduced proteolytic activity compared to the upper gastrointestinal tract, it is colonized with a large population of bacteria which secrete bacterial proteases.

Co-administration of protease inhibitors (PI) to inhibit enzymatic degradation of protein payload is one key strategy which is employed for oral delivery of protein/peptide drugs.

The aim of this study was to determine the stability of adalimumab in the presence of colonic microbiota of a healthy individual and three Crohn's Disease patients when administered as such, when co-administered with HPBCD, and when co-administered with three protease inhibitor (PI) mixes, whether or not combined with HPBCD.

#### I-II Material and Methods

An overview of the experimental design and set-up is shown in Figures 10 and 11.

HPBCD and protease inhibitor mixes were added to a carbohydrate-supplemented nutritional medium. Then, 10% (v/v) of a cryopreserved 7.5% (w/v) fecal inoculum of the aforementioned donors was added to the reactors as microbial source. Finally, the mAb was spiked from a Tris-Acetate stock solution into the respective reactors, bringing the total volume in the reactors to 50 ml and the mAb concentration to 1 mg/mL. Reactors were incubated for 48h at 37°C, under continuous (mild) shaking and anaerobic atmosphere.

Samples for mAb quantification were collected and centrifuged at 7690 xg for 5 min to remove bacteria. The supernatant was flash frozen in liquid nitrogen and stored in an ultra-freezer (-80 °C) until analysis. Prior to HPLC analysis, all samples were thawed at room temperature and purified with Amicon® Ultra-2 50K filters to discard LMW compounds. In order to do so, samples were centrifuged for 5 min at 27760 xg and supernatants filtered with 0.2 $\mu$m filters. Samples were

then fivefold diluted in chilled PBS and gently mixed to homogenize the samples. A volume of 1500 µL sample was brought on the Amicon® Ultra-2 50K filters and samples were centrifuged at 7500 xg for 20 min. The filtrate was discarded, and a washing step was performed by loading 1500 µL chilled PBS on the Amicon® Ultra-2 50K filters, followed by a centrifugation step at 7500 xg for another 20 min. Filtrate was removed and the filters were inverted to recover the mAb by means of 2 min centrifugation at 1000 xg. The obtained volume was pipetted into a new Eppendorf, was tenfold diluted in chilled PBS, and further (threefold) diluted in eluent. The obtained solution was subjected to a final centrifugation step of 10 min at 18320 xg. A volume of 200 µL supernatant was pipetted into the insert of an HPLC vial to perform HPLC analysis.

Size Exclusion Chromatography (SEC) was performed to quantify the mAb in each sample. Ultra-High-Performance Liquid-Chromatography (UHPLC) coupled to a Photo Diode Array (PDA) detector was selected as detection method. An overview of the chromatographic parameters is given in Figure 12.

The PathHunter® adalimumab Bioassay Kit (Eurofins DiscoverX) was used according to manufacturer's instructions for measurement of adalimumab biological activity.

Results are showed in Figure 13 and 14.

### I-III Results and discussion

The third patient was excluded due to a problem during the fermentation phase.

Adalimumab alone was degraded to an average of 55% remaining after 24 h of colonic incubation (Figure 13). In the presence of HPBCD, up to 75% of adalimumab was detected after 24 h of colonic incubation. The three PI mixes (containing known protease inhibitors) did not further improve the stability of adalimumab compared to the stability observed with HPBCD. Combining PI mixes and HPBCD did not improve adalimumab stability further. HPBCD was able to ensure adalimumab stability in a colonic environment to a similar level as established protease inhibitors.

Adalimumab recovered after 24 h simulated colonic incubation retained biological activity (Figure 14).

Adalimumab is susceptible to microbial degradation in the colonic environment. Addition of HPBCD or protease inhibitors improved mAb stability in terms of its physical stability and biological activity. HPBCD is able to ensure adalimumab stability in the colonic environment to a similar level as established protease inhibitors.

### J- Local administration of adalimumab, alone or in combination with hydroxypropyl-beta-cyclodextrin (HPBCD)

#### J-I Introduction

In this study, adalimumab was used as the therapeutic protein model. Adalimumab is the antibody that is found in the commercial product Humira® (ABBVIE). The adalimumab used was produced in-house, as disclosed in previous sections.

The purpose of this study was first to determine the performance of local administration (intra-rectal (IR)) of adalimumab instead of subcutaneous (SC) administration, to verify whether oral administration of adalimumab could be successful.

The second objective was to determine the performance of a combination of HPBCD with adalimumab, the minimum therapeutically effective dose, safety of the dosages, and to verify the obtaining of a dose-response effect.

To this end, local administration of an adalimumab formulation according to the disclosure was compared with Humira® administered subcutaneously (indicated route of administration of Humira®) or locally (intra-rectal (IR)).

#### J-II Pre-test study

The aim of the pre-test study was to compare the therapeutic properties of adalimumab manufactured in-house (referred to as "ADM" in Examples section J) to the benchmark biologic Humira® in use in inflammatory bowel disease (IBD) patients and to find the best maximal dosage. The tested products were administered by IR route or SC injection at 2 dosages in the Gold standard model of colitis induced by 2,4,6-trinitrobenzene sulfonic acid (TNBS) in Sprague-Dawley rats.

#### J-II, 1. Protocol

The anti-inflammatory effects were evaluated in colitic rats receiving the different treatments and compared to those observed in TNBS rats receiving only the vehicle (colitic control rats). Assessment of inflammation was performed at macroscopic level using the multiparametric Wallace's score (Wallace JL, Keenan CM, Gale D, Shoupe TS. Exacerbation of experimental colitis by nonsteroidal anti-inflammatory drugs is not related to elevated leukotriene B4 synthesis.

Gastroenterology. 1992 Jan;102(1):18-27).

*J-II, 1.1. Animal <u>model</u>*

The rat was chosen as the animal model, as it is more resistant to colitis induced by TNBS compared to mice. The rats used were males having a weight of about 100-125 g. They were 4 weeks old when they arrived in the laboratory, and they were acclimated to the study conditions for a period of at least 7 days before the beginning of the pre-treatment period.

*J-II, 1.2. <u>Test items</u>*

50 rats were allocated randomly to 5 groups, as shown in Table 4.

[Table 4]

| Test items | Antibody dosage* | Route | Number of rats Igroups |
|---|---|---|---|
| Colitic control IR | Not applicable | IR** | 10 |
| Colitic rats - Humira® 15mg/kg SC | 15mg/kg | SC | 10 |
| Colitic rats - Humira® 15mg/kg IR | 15mg/kg | IR | 10 |
| Colitic rats - Humira® 30mg/kg IR | 30mg/kg | IR | 10 |
| Colitic rats - ADM 15mg/kg IR | 15mg/kg | IR | 10 |
| *the quantity of antibody is in dry weight, ** the product administered is histidine buffer | | | |

IR administration was performed by IR instillation, and SC administration by SC injections.

For the ADM treated rats: ADM was formulated in the same buffer as used in HUMIRA® based on a mean weight of rats of 125g to obtain the control dose adalimumab 15 mg/Kg in a volume of 250 $\mu$L for IR administration. The composition was thus the following: 50 mg/mL ADM, 6.1625 mg/mL NaCl, 0.8625 mg/mL Monobasic sodium phosphate dihydrate, 1.525 mg/mL Dibasic sodium phosphate dihydrate, 0.3 mg/mL Sodium citrate, 1.3 mg/mL Citric acid monohydrate, 12 mg/mL Mannitol, 1 mg/mL Polysorbate 80, NaOH to adjust to pH 5.2.

The rats were treated by administration of a volume of 250 $\mu$L of item to be tested every 2 days from D-5 until euthanasia at D4 (Figure 15). After completion of the treatment, animals were euthanized i.e., 4 days after colitis induction.

The colitic control rats received a histidine buffer by IR route because this buffer was the one to be used in the following studies using ADM freeze-dried with HPBCD.

*J-II, 1.3. <u>Colitis induction</u>*

Colitis was induced by an IR injection of 250 $\mu$L TNBS (80 mg/kg in 40% EtOH) at 8 cm from the anus using a catheter (Centracath, ref. 1230.20, 8 cm of length) (D0, Figure 15). The TNBS solution at 80 mg/kg is prepared based on the mean body weight of rats. Evaluation of the intensity of colitis was performed 4 days later. Animals were sacrificed 4 days after TNBS administration (D4, Figure 15).

*J-II, 1.4. <u>Clinical Examination and Macroscopic assessment of inflammation</u>*

Mortality was monitored every day from D-5, until euthanasia at D4. The body weight of each animal was recorded every day from D-5 to D4. The rate of diffused rats was also recorded. Indeed, diffusion is an event classically encountered in the model of colitis induced by a high dose of TNBS (80mg/kg). The diffusion of TNBS from the colon to other organs (yellow-colored organs) occurs as a result of a higher permeability of the colonic wall leading to peritonitis.

Macroscopic assessment of colitis was performed independently by two operators to validate the score. If discrepancy was observed between the two obtained scores, a third evaluation was performed. The colon of each rat was examined to evaluate the macroscopic lesions according to the Wallace criteria. The Wallace score rates macroscopic lesions on a scale from 0 to 10 (Table 5), and this score is based on features reflecting inflammation, such as hyperemia, thickening of the bowel, and extent of ulceration.

[Table 5] Wallace score

| Score | Criteria of macroscopic evaluation |
|---|---|
| 0 | No inflammation |
| 1 | Hyperemia without ulcerations |
| 2 | Hyperemia with thickening of the mucosa without ulcerations |
| 3 | 1 ulceration without thickening of the colonic wall |
| 4 | 2 or more ulcerative or inflammatory sites |
| 5 | 2 or more ulcerative or inflammatory sites with an extent of > 1 cm |
| 6 | Ulcerative or inflammatory site > 2 cm |
| 7 | Ulcerative or inflammatory site > 3 cm |
| 8 | Ulcerative or inflammatory site > 4 cm |
| 9 | Ulcerative or inflammatory site > 5 cm |
| 10 | Ulcerative or inflammatory site > 6 cm |

*J-II, 1.5. Blood and tissue sampling*

Serum from each animal was collected at the time of euthanasia by cardiac puncture, placed in a 1.1ml Z-Gel micro tube (Sarstedt). Tubes were inverted 5 times and samples were then allowed to clot for 1 hour at ambient temperature. Tubes were centrifuged at 2300XG for 10 minutes at 20°C. Serum (supernatant) was collected into a fresh pre-chilled.
Samples from distal colon were divided in 2 parts and snap-frozen (one part for protein analysis and the other one for mRNA extraction) a piece of liver, spleen and MLNs (when possible) were snap-frozen.

*J-II, 1.6. Statistical Analysis*

All comparisons were analyzed using the Permutation Test for two independent samples, the most powerful statistical test adapted to a small number of samples. Statistics were calculated using the StatXact software (Cytel Inc, Cambridge, MA, USA). Differences were considered statistically significant if the p-value was <0.05. The comparisons were done against the TNBS control group receiving the vehicle (colitic control rats).

**J-II, 2. Results and discussion**

***J-II, 2.1. Mortality***

No colonic lesions were observed in rats having a peritonitis due to TNBS diffusion and therefore these "diffused rats" characterized by the absence of colonic lesions and presence of TNBS in other organs (yellow color) indicating diffusion were excluded from analysis (n=6/50). No mortality was recorded in any group of colitic rats, and we did not observe significant differences regarding the rate of diffusion. This result indicates the safety of subcutaneous or intrarectal administration of the tested products at the dosage of 15 or 30mg/kg every two days even under inflammatory conditions.

***J-II, 2.2. Body weight evolution***

The main interest of this marker in this study was to demonstrate the homogeneity of groups before colitis induction and to demonstrate the lack of side effects of the tested products.
The body weight results (data not shown) indicate that Humira® and ADM administered at the dosage of 15mg/kg or 30mg/kg have no side effects even under inflammatory conditions when administered by subcutaneous or intrarectal route. Moreover, Humira® or ADM at the dosage of 15mg/kg by IR or SC injections may improve the body weight gain of colitic rats.

***J-II, 2.3. Post-mortem examination (Figure 16)***

Results are expressed as mean ± SEM. A significant decrease of the colon weight was observed in the groups of colitic rats receiving Humira® or ADM by IR route, at the dosage of 15mg/kg compared to control colitic rats (Figure 16). This result indicates that Humira® 15mg/kg IR and ADM 15mg/kg IR decrease significantly the edema induced by the acute colitis. This result was confirmed by the calculation of the ratio of the weight/lenght of the colon.

*J-II, 2.4. Macroscopic evaluation (Wallace score) (Figure 17)*

Intensity of colonic inflammation and lesions was evaluated at macroscopic level according to the validated Wallace criteria (Table 5). Results are expressed as mean ± SEM score (Figure 17).
As expected, a high score of inflammation was observed in colitic control rats. They suffered from severe colitis even 4 days after colitis induction with an ulcerative or inflammatory site superior to 2 to 3 cm.
A significant decrease of the Wallace score was observed in colitic rats treated with Humira® at 15mg/kg administered by IR route, when compared to colitic control rats, corresponding to a percentage of improvement of inflammatory lesions of 30%. On the other hand, no significant decrease of the inflammatory lesions was observed in the group of colitic rats treated with Humira® at 30mg/kg administered by IR. This was expected because a too high antibody dosage may cause to reverse TNF-α signaling, and thus to a loss of antibody activity.
A higher significant anti-inflammatory effect was recorded in colitic rats treated with ADM at 15mg/kg by IR route, corresponding to an improvement of the inflammatory lesions of 43%.
Altogether these results validate that the Gold standard model of colitis induced by TNBS is suitable to compare and evaluate the anti-inflammatory properties of adalimumab. It was further noted that ADM seems to exert higher anti-inflammatory effects compared to Humira® at the same dosage of 15mg/kg every two days by IR.

**J-III Evaluation of a combination of adalimumab with hydroxypropyl-beta-cycodextrin (HPBCD)**

The aim of the study was to compare the therapeutic properties of 4 dosages of adalimumab formulated with HPBCD (hereinafter referred to as "ADM + HPBCD") to Humira® and to 5-ASA (the active principle of Pentasa®), two benchmark drugs used in patients suffering from IBD, administered at their optimal dosage. The tested products were administered by IR route in the Gold standard model of colitis induced by TNBS in Sprague-Dawley rats.

**J-III, 1. Protocol**

Assessment of inflammation was performed at macroscopic level using the multiparametric Wallace score and at histological level according to Ameho criteria (Ameho C.K.et al. Prophylactic effect of dietary glutamine supplementation on interleukin 8 and tumor necrosis factor α production in trinitrobenzene sulfonic acid induced colitis. Gut. 1997;41:487-493.).

*J-III, 1.1. Animal model*

The animal model and adaptation were as described in section J-II, 1.1.

*J-III, 1.2. Test items*

75 rats were allocated randomly to 8 groups, as shown in Table 6.

[Table 6]

| Test Items | Antibody dosage* | Route | Number of rats /groups |
|---|---|---|---|
| Healthy control rats | NA | IR** | 5 |
| Colitic control rats | NA | IR** | 10 |
| Colitic rats - ADM 2.5mg/kg + HPBCD | 2.5mg/kg | IR | 10 |
| Colitic rats - ADM 5mg/kg + HPBCD | 5mg/kg | IR | 10 |
| Colitic rats - ADM 10mg/kg + HPBCD | 10mg/kg | IR | 10 |
| Colitic rats - ADM 15mg/kg + HPBCD | 15mg/kg | IR | 10 |
| Colitic rats - 5-ASA 30mM | 150mg/kg | IR | 10 |

(continued)

| Test Items | Antibody dosage* | Route | Number of rats /groups |
|---|---|---|---|
| Colitic rats - Humira® 15mg/kg | 15mg/kg | IR | 10 |
| * the quantity of antibody is in dry weight, ** the product administered is histidine buffer | | | |

For the rats treated with ADM + HPBCD, the infusion was prepared from a freeze-dried solid product obtained from a solution comprising 12.5 mg/mL of adalimumab, 62.5 mg/mL of HPBCD (KLEPTOSE® HPB, ROQUETTE FRERES) and 3.78 mg/mL of L-histidine/ Histidine HCl obtained according to section F-II.

For the adalimumab dosage of 15 mg/kg, ADM + HPBCD powder was reconstituted with 2.347 mL of purified water to make up to 2.5 mL of solution. Reconstituted solution contained 12.5 mg/mL of adalimumab - Assuming 250 g max rat weight, adalimumab dose = 3.75 mg = 300 $\mu$L of 12.5 mg/mL reconstituted solution. Other dosages were prepared by diluting this stock solution.

Administration of ADM + HPBCD and Humira® was performed by IR instillation as described in section J-II, 1.2.

Administration of 5-ASA was performed by IR instillation. The rats were treated by administration of a volume of 250 $\mu$L of solution, at a dosage of 30 mM (equivalent to 150 mg/kg) to be tested every day from D-5 until euthanasia at D4 (Figure 15).

*J-III, 1.3. Colitis induction*

Colitis induction was performed as described in section J-II, 1.3.

*J-III, 1.4. Clinical Examination and Macroscopic assessment of inflammation*

Clinical examination and macroscopic assessment of inflammation were performed as described in section J-II, 1.4.

*J-III, 1.5. Histological assessment of inflammation*

A colon specimen located 2 cm above the anal canal was used for histological evaluation according to Ameho criteria. This scoring on a scale from 0 to 6 takes into account the degree of inflammation infiltrate, the presence of erosion, ulceration, or necrosis, and the depth and surface extension of lesions (Table 7).

[Table 7] Ameho score

| Score | Criteria of histologic evaluation |
|---|---|
| 0 | No alterations |
| 1 | Middle mucosal and/or sub-mucosal inflammatory infiltrates with oedema. Few mucosal erosions. Integrity of the muscularis mucosae |
| 2 | Same criteria as score 1 but >50% of the section |
| 3 | Large inflammatory infiltrate with ulceration area through all the colonic wall |
| 4 | Same criteria as score 3, >50% of the section |
| 5 | Wide ulcerations with cellular necrosis |
| 6 | Wide ulcerations with cellular necrosis >50% of the section |

*J-III, 1.6. Blood and tissue sampling*

Blood and tissue sampling were performed as described in section J-II, 1.5.

*J-III, 1.7. Statistical analysis*

Statistical analysis was performed as described in section J-II, 1.6.

**J-III, 2. Results and discussion**

*J-III, 2.1. Mortality*

The "diffused rats" characterized by the absence of colonic lesions and presence of TNBS in other organs (yellow color) indicating diffusion were excluded from analysis (n=7/75).

No significant difference regarding the rate of diffusion and mortality was observed in the different groups of rats receiving either the different treatment. This result indicates the safety of intrarectal administration of the tested products at the dosage of 2.5, 5, 10 or 15mg/kg every two days even under inflammatory conditions.

*J-III, 2.2. Body weight evolution*

Body weight results (data not shown) indicate the safety of topical administration of ADM at the different tested dosages under physiological or inflammatory conditions.

*J-III, 2.3. Post-mortem Examination (data not shown)*

The results were similar to what we observed in the pre-test study (section J-II, 2.3).

*J-III, 2.4. Macroscopic evaluation (Wallace score) (Figure 18)*

Intensity of colonic inflammation and lesions was evaluated at macroscopic level according to the validated Wallace criteria (Table 5). Results are expressed as mean $\pm$ SEM score (Figure 18).

A significant decrease of the Wallace's score was observed in colitic rats receiving the Humira® treatment at the adalimumab dosage of 15mg/kg administered in topical route by intrarectal instillation, the positive control, when compared to colitic control rats, corresponding to a percentage of improvement of inflammatory lesions of 24%. A significant decrease of the inflammation was also observed with the second positive control, the 5-ASA administered by IR at its optimal dosage of 150mg/kg (30mM), decreasing by 34% the level of inflammation at the macroscopic level. These results validate the model and the study.

An important trend to decrease by 24% the level of the inflammation at the macroscopic was observed in the group of colitic rats receiving ADM 10mg/kg + HPBCD. The efficacy of ADM 10mg/kg + HPBCD what similar to that observed when using Humira® at its optimal adalimumab dosage of 15mg/kg.

A significant anti-inflammatory effect was recorded in colitic rats receiving ADM 15mg/kg + HPBCD administered by intrarectal instillation (topical administration), corresponding to an improvement of the inflammatory lesions of 27%.

This study confirms that ADM + HPBCD exerts higher anti-inflammatory effects compared to Humira®.

*J-III, 2.5. Histological assessment of inflammation (Figure 19)*

Evaluation of inflammation and colonic lesions at histological level was performed according to the validated score of Ameho (as illustrated in Table 7). Results are expressed as a mean $\pm$ SEM score (Figure 19).

Regarding the reference product i.e., Humira® administered at its optimal dosage, no improvement was observed at the histological level compared to colitic control rats. Indeed, an improvement of 7% of the inflammatory lesions was measured. This result can be explained by the fact that the evaluation was performed at the peak of inflammation and wound-healing process induced by the treatment is not visible at this early stage.

An important trend to improve the inflammatory lesions was observed at the histological level in the colitic rats receiving the second positive control, the 5-ASA able to improve by 24% the intensity of inflammation.

An important trend of inducing a decrease (of 21%) of the inflammatory lesions, similar to that induced by 5-ASA treatment, was observed in the groups of colitic rats receiving ADM 10mg/kg + HPBCD or ADM 15mg/kg + HPBCD.

These results at the histological level are in good correlation with those obtained at the macroscopic level indicating that ADM + HPBCD may have more important anti-inflammatory effects compared to Humira® (not containing HPBCD) administered at its optimal dosage by IR instillations in colitic rats. The dosage of 10mg/kg exerts also important anti-inflammatory effects.

**J-IV Assessment of the therapeutic properties of local administration of adalimumab and hydroxypropyl-beta-cyclodextrin (HPBCD)**

The therapeutic properties of ADM + HPBCD treatment were also evaluated by measuring the level of cell infiltrates (Colonic level of Lipocalin-2) at the protein level. The gene level expression of genes involved in the reinforcement of the intestinal barrier (ZO-1, Occludin and MUC2) were quantified at the mRNA level using the qRT-PCR method. Immunohistochemical staining was performed to evaluate cell proliferation (PCNA IHC staining) and apoptosis (Tunel IHC) to determine the ADM + HPBCD properties on wound-healing properties of the colonic mucosa.

**J-IV, 1. Protocol**

*J-IV, 1.1. <u>Impact on inflammation: level of cell infiltrates (Colonic level of Lipocalin-2 (Lcn-2) at the protein level</u>*

The level of Lipocalin-2 (Lcn-2), a marker of neutrophil infiltration (enzyme contained in polymorphonuclear neutrophil primary granules) was quantified by ELISA (Abcam, Rat Lipocalin-2 ELISA kit ref ab239422) on a piece of distal colon taken off at euthanasia (D4). Colon pieces were homogenized (50 mg/ml) in Tris-HCl buffer containing protease inhibitors (Sigma-Aldrich) in a Precellys homogenizer using ceramic beads (1.4 & 2.8 mm) (Bertin, France). The samples were centrifuged for 20 min and the supernatant was frozen at -80°C until the assessment using ELISA kits and according to the manufacturer's recommendations. The quantification of Lcn 2 marker was performed on colon samples from control rats, colitic rats receiving the vehicle and colitic rats receiving 15mg/kg ADM + HPBCD by IR.

*J-IV, 1.2. Evaluation <u>of gene expression by quantitative RT-PCR (qRT-PCR) in the colonic wall</u>*

Gene expression was assessed by qRT-PCR using the primers as shown in Table 8. Briefly, total RNA was extracted with a Nucleospin RNA kit (Macherey-Nagel, Hoerdt, France). After RNAse inactivation, the total RNA was cleaned of traces genomic DNA via a DNAse treatment and eluted in RNAse-free, DEPC-free water. The purity of the RNA was evaluated by UV spectroscopy on a Nanodrop system from 220 to 350 nm. 1 μg of total RNA was used to perform a qRT-PCR by using LightCycler FastStart DNA Master SYBR Green I from Roche Diagnostics (Indianapolis, IN) according to the manufacturer's protocol. For each reaction, a critical threshold cycle (Ct) value, indicating the cycle number at which the DNA amplification was determined. Relative gene expression value was calculated as $E=2-\Delta Ct$, where $\Delta Ct$ is the difference in crossing points between GAPDH and each gene.

The expression at the mRNA level of 3 genes involved in the intestinal permeability and one housekeeping gene (GAPDH) were measured. The primers used had the sequences as showed in Table 8.

[Table 8] <u>Primer sequences</u>

| Rat genes | Primer sequences (5' → 3') |
|---|---|
| GAPDH | F:5'-CTG-TTC-TAG-AGA-CAG-CCG-CAT-CT-3' <br> R: 5'-ACA-CCG-ACC-TTC-ACC-ATC-TTG-3' |
| MUC2 | F:5'-gCC-AgA-TCC-CgA-AAC-CA-3' <br> R: 5'-TAT-Agg-AqT-CTg-ggC-AgT-CA-3' |
| ZO-1 | F: 5'-CggAACTATgACCATCgCCATC-3' <br> R: 5'-gCCTgTACCTgTTgTgCACC-3' |
| Claudin-2 | F: 5'-CgAgAAAgAACAgCTCCgTTT -3' <br> R: 5'-TTCgCTTgTCTTTTggCTgC-3' |

*J-IV, 1.3. <u>Anti-inflammatory effect: evaluation by immunohistostaining of colonic epithelial cells proliferation and apoptosis</u>*

Tissues (colon) were immediately placed into 4% of paraformaldehyde solution for histological studies. Evaluation of epithelial cell proliferation in the colonic mucosa was performed by IHC using a rabbit polyclonal anti-PCNA antibody (1mg/ml, Biotechne, NB100-456). Tissues were incubated with the primary anti-PCNA antibody at the concentration of 1/1000e diluted in PBS+ 0.5% of Tween 20 and 3% of goat serum overnight at 4°C. A secondary antibody (anti-goat rabbit Alexa-fluor 488, 1/100e, 1 h) was used. Hoescht staining was used to stain the cell's nucleus.

Tunel immunohistostaining to detect the level of apoptosis was performed using the "In situ cell death detection kit, TMR red" (Roche, Ref 12156792910) according to the manufacturer's recommendations.

**J-IV, 2. Results <u>and Discussion</u>**

*J-IV, 2.1. <u>Impact on inflammation: level of cell infiltrates (Colonic level of Lcn-2) at the protein level (Figure 20)</u>*

Lcn-2, and its human counterpart Neutrophil gelatinase-associated lipocalin (NGAL), belong to a family of small, secreted proteins expressed by a variety of cells, the richest source being neutrophils. Systemic and fecal upregulation of Lcn-2 have been widely demonstrated in various murine models of colitis (Hsieh H et al; Toxicologic Pathology, 2016; Chassaing B et al, PlosOne 2012). In addition, human NGAL has been reported to be increased in patients with ulcerative colitis.

Samples from colitic control rats receiving the vehicle (samples pooled from previous studies (sections J-II and J-III), colitic rats receiving ADM 15mg/kg + HPBCD (samples from previous study (section J-III)), colitic rats receiving ADM 10mg/kg + HPBCD (samples from previous study (section J-III)), and Humira® at an adalimumab dosage of 15mg/kg were used for Lcn-2 quantification. Results are expressed in pg/mg of tissue as mean $\pm$ SEM (Figure 20).

A significant increase of the level of Lcn-2 marker was observed in the colon of colitic rats receiving vehicle, when compared to healthy control rats. This result indicates the presence of a high level of inflammatory cells infiltrates due to the severe colitis induced by TNBS IR instillation.

No significant decrease of the inflammatory cells infiltrates was observed in the group of colitic rats receiving Humira® at its optimal dosage compared to colitic control rats corresponding to a decrease of 7% of the inflammatory cell infiltrates in the colon.

A high trend to decrease the inflammatory cell's infiltrates was recorded in the colon of colitic rats receiving ADM 10mg/kg + HPBCD or ADM 15mg/kg + HPBCD (corresponding to a decrease of 40% and 43% respectively).

These results correlate with those obtained at the macroscopic and histological levels: topical administration of ADM 10mg/kg + HPBCD or of ADM 15mg/kg + HPBCD significantly decreases the inflammation. ADM + HPBCD treatment may reduce the inflammation to a greater extent than HUMIRA® (which does not contain HPBCD).

*J-IV, 2.2. Evaluation <u>of the Evaluation of the effects of ADM + HPBCD Treatment on the expression of genes involved in the reinforcement of the intestinal barrier (Figure 21)</u>*

During severe inflammatory episodes, an increase in intestinal permeability is observed. Increased intestinal permeability is also associated with increased visceral pain. The effect of different treatments was assessed on the reinforcement of the intestinal barrier by measuring MUC2 expression at mRNA level.

The aim of this study was to assess whether the products tested alone or in combination can strengthen the intestinal barrier by modulating mucin (MUC2) expression at mRNA level. A layer of mucus covers all mucosal surfaces, forming an important barrier. Mucus is a viscous gel composed mainly of water (90% or more), with small amounts of salts, carbohydrates, lipids and mucins. This complex, viscous mucus network plays a key role in protecting the intestinal mucosa from pathogens. The mucus layer throughout the digestive tract also acts as a chemical and physical barrier to drug absorption.

Proteins of the claudin family (including zonula occludins (ZO) and MAGUK family proteins) are essential components of tight junctions and thus plays an important role in intestinal permeability.

In this study the effect of ADM + HPBCD treatment was evaluated on the level of expression of ZO-1, Occludin and Muc2 at the mRNA level. Samples from control colitic rats, colitic rats receiving ADM 15mg/kg + HPBCD, colitic rats receiving ADM 10mg/kg + HPBCD, and Humira® at an adalimumab dosage of 15mg/kg were used to evaluate the gene expression at the mRNA level. (Figure 21, results expressed as mean $\pm$ SEM).).

In control colitic rats, compared to control healthy rats, no significant modification of the level of expression of tight junction proteins (ZO-1 and Occludin) was recorded. A trend to increase the expression of Muc2 at the mRNA level was observed due to the severe inflammation induced by TNBS.

A significant increase of ZO-1 expression at the mRNA level was observed in colitic rats receiving ADM + HPBCD compared to control colitic rats. A significant increase of the level of expression of Occludin was observed in colitic rats receiving ADM 10mg/kg + HPBCD, compared to control colitic rats. Moreover, a trend to decrease the level of Muc2 was observed at the mRNA level with ADM 15mg/kg + HPBCD treatment.

Altogether, these results indicate that ADM + HPBCD local treatment, by decreasing the level of inflammation, plays a role in the reinforcement of the intestinal barrier.

*J-IV, 2.3. Evaluation <u>of ADM + HPBCD treatment on cellular proliferation and apoptosis</u>*

The level of intestinal cell apoptosis and proliferation was evaluated in the colon section using immunohistochemistry staining using respectively the Tunel method for the apoptosis evaluation and the immunohistochemistry staining using PCNA antibody for the cell proliferation. After immunostaining, a software quantifies the number of positive stained cells and the total number of cells for the studied section of the colon.

*J-IV, 2.3.1. Evaluation of ADM + HPBCD Treatment on cell proliferation (Figure 22)*

In physiological conditions, a proliferation of intestinal epithelial cells (IECs) is observed in the crypt of the mucosa as observed in healthy control rats without inflammation (Figure 22, results expressed as mean $\pm$ SEM).

As expected, the severe inflammation caused by TNBS destroying the mucosa until inducing area of necrosis induced a significant decrease of the level of proliferative IECs in the crypts of control colitic rats compared to control healthy rats.

ADM + HPBCD treatment restored the proliferation of IECs in a dose dependent manner. Indeed, ADM 10mg/kg + HPBCD treatment administered by IR instillation induced a significant increase of 54% of the IECs proliferation, compared to control colitic rats. The increase of the cell proliferation index was increased up to 75% in colitic rats receiving ADM 15mg/kg + HPBCD. Similar results were observed in the colitic rats treated with Humira® at an adalimumab dosage of 15mg/kg, showing also an increase of the index of cell proliferation of 75% compared to control colitic rats.

These results correlate with those observed at the macroscopic and histological level confirming that due to the important anti-inflammatory properties, local adalimumab treatment restores the level of IECs proliferation in colitic rats and allows the wound-healing process.

*J-IV, 2.3.2. Evaluation of ADM + HPBCD treatment effects on cell apoptosis (Figure 23)*

In physiological conditions, a very low level of apoptosis (red staining) was observed (healthy control rats, Figure 23, results expressed as mean $\pm$ SEM).). On the other hand, a significant increase of the level of apoptotic cells was recorded in control colitic rats.

ADM + HPBCD treatment significantly decreased the level of apoptosis at the two tested adalimumab dosages of 10mg/kg and 15mg/kg administered by IR instillation (about 90% decrease of the apoptosis) compared to control colitic rats.

In the colitic rats treated with Humira® at the adalimumab dosage of 15mg/kg, a significant but lower decrease of 78% of the apoptosis was observed in their colonic inflamed mucosa compared to control colitic rats.

This result confirms the efficacy of adalimumab local treatment for significantly reducing the level of apoptotic cells. Formulating adalimumab with HPBCD may allow to reduce the level of apoptotic cells even more (cf., ADM + HPBCD treatment in comparison with HUMIRA® treatment).

**Claims**

1. **An** oral colon targeted tablet comprising a tablet core coated with a film-coating composition, said tablet core comprising a therapeutic protein powder, said therapeutic protein powder being obtained by drying a liquid comprising a therapeutic protein and a substituted cyclodextrin, and said film-coating coating composition comprising a water-insoluble polymer and a soluble fiber.

2. The oral colon targeted tablet of claim 1, wherein said substituted cyclodextrin is substituted with alkyl groups and/or hydroxyalkyl groups and/or sulfoalkyl group.

3. The oral colon targeted tablet of claim 2, wherein said substituted cyclodextrin is substituted with alkyl groups and/or hydroxyalkyl groups and/or sulfoalkyl group, having 1 to 5 carbon atoms.

4. The oral colon targeted tablet of any of claims 1 to 3, wherein said substituted cyclodextrin is selected from hydroxypropyl-cyclodextrin, methyl-cyclodextrin, sulfobutyl ether cyclodextrin, or from a mixture thereof.

5. The oral colon targeted tablet of any of claims 1 to 4, wherein said therapeutic protein is an antibody.

6. The oral colon targeted tablet of any of claims 1 to 5, wherein said therapeutic protein is a TNF-$\alpha$ inhibitor.

7. The oral colon targeted tablet of any of claims 1 to 6, wherein said water-insoluble polymer is selected from ethylcellulose, cellulose derivatives, acrylic and/or methacrylic ester polymers, polymers or copolymers of acrylate or methacrylate polyvinyl esters, starch derivatives, polyvinyl acetates, polyacrylic acid esters, butadiene styrene copolymers methacrylate ester copolymers, cellulose acetate phtalate, polyvinyl acetate phtalate, shellac, methacrylic acid copolymers, cellulose acetate trimellitate, hydroxypropyl methylcellulose phtalate, zein, starch acetate, or from any mixture thereof.

8. The oral colon targeted tablet of claim 7, wherein said water-insoluble polymer is ethylcellulose.

9. The oral colon targeted tablet of any of claims 1 to 8, wherein said soluble fiber is selected from xylooligosaccharides, inulin, oligofructoses, fructo-oligosaccharides (FOS), lactulose, galactomannan, suitable hydrolysates thereof, indigestible polydextrose, indigestible dextrins, partial hydrolysates thereof, trans-galacto-oligosaccharides (GOS), xylooligosaccharides (XOS), acemannans, lentinans, beta-glucans, partial hydrolysates thereof, polysaccharides-K (PSK), indigestible maltodextrins, partial hydrolysates thereof, or from any mixture thereof.

10. The oral colon targeted tablet of claim 9, wherein said soluble fiber is selected from a soluble indigestible maltodextrin, a soluble indigestible dextrin, or from a mixture thereof.

11. The oral colon targeted tablet of any of claims 1 to 10, wherein the water-insoluble polymer:soluble fiber dry weight ratio in the film-coating composition is selected from (1:1) to (8:1).

12. A process for preparing an oral colon targeted tablet according to any of claims 1 to 11 comprising:

a step (a) of obtaining said tablet core; and,
a step (b) of coating said tablet core with said film-coating composition.

13. An oral colon targeted tablet obtainable from the process of claim 12.

14. An oral colon targeted tablet according to any of claims 1 to 11, or an oral colon target tablet according to claim 13, for use as a medicament.

15. The oral colon targeted tablet for use according to claim 14, for the treatment of an inflammatory bowel disease.

[Fig. 1] **Drug release from theophylline tablets coated to 15% and 30% weight gain in pH transition media simulating gastric and small intestinal phases (Examples section A).**

theophylline Release

[Fig. 2] **igG recovery without or with a stabilizer HPBCD (Examples section B).**

**Figure 2.a) % monomer recovery and aggregate level of human plasma IgG without stabilizing excipients after each process step of tableting, simulated coating and simulated film curing .**

Human IgG without stabilizing excipients

**Figure 2.b) % monomer recovery and aggregate level of human plasma IgG freeze-dried with 12.5% and 25% HPBCD solution (corresponding to IgG:HPBCD (1:10) and (1:20) weight ratios) after each process step of freeze drying, tableting, simulated coating and simulated film curing.**

Human IgG freeze-dried with HPBCD

[Fig. 3] % monomer recovery and aggregate level of adalimumab freeze-dried in citrate buffer with 12.5% and 25% HPBCD solution (corresponding to adalimumab:HPBCD (1:10) and (1:20) weight ratios) after each process step of freeze drying, tableting, and simulated tablet coating (Examples section C).

[Fig. 4] % monomer recovery of adalimumab freeze-dried in histifine buffer with 12.5% and 25% HPBCD solution (corresponding to adalimumab:HPBCD (1:10) and (1:20) weight ratios) after each process step of freeze drying, tableting, simulated tablet coating and curing (Examples section D).

[Fig. 5] % monomer recovery of adalimumab freeze-dried with 6.25% and 12.5% HPBCD solution (corresponding to adalimumab:HPBCD (1:5) and (1:10) weight ratios) after each process step of freeze drying, tableting, tablet coating and curing (Examples section F).

[Fig. 6] **TNF-α binding activity of adalimumab samples determined by ELISA after freeze drying, blending and tableting; in comparison to monomer recovery by size-exclusion chromatography (Examples section F).**

[Fig. 7] **Biological activity of adalimumab samples determined by cell-based assay after the process steps of freeze drying, tableting, tablet coating and curing; in comparison to monomer recovery by size-exclusion chromatography (Examples section F).**

[Fig. 8] **Human plasma IgG release from tablets coated with ethylcellulose:NUTRIOSE® blend under conditions simulating transit through the gastrointestinal tract, with fecal samples from IBD patients (Examples section G).**

Ethylcellulose:NUTRIOSE® - ratio (4:1)

Ethylcellulose:NUTRIOSE® - ratio (3:1)

[Fig. 9] **Adalimumab release from tablets coated with Ethylcellulose:NUTRIOSE®
blend under conditions simulating transit through the gastrointestinal tract with
fecal samples from IBD patients (Examples section H).**

Ethylcellulose:NUTRIOSE® - ratio (4:1), 25% coating

Ethylcellulose:NUTRIOSE® ratio (3:1), 30% coating

[Fig. 10] **Experimental design to test the effects of protein stabilizer and different protease inhibitor mixes on the stability of adalimumab in the presence of colonic microbiota (Examples section I).**

[Fig. 11] **Experimental set-up comprising one heathy donor and three donors with Crohn's Disease (Examples section I).**

[Fig. 12] **Chromatographic parameters for mAb detection (Examples section I).**

| UHPLC system | Waters ACQUITY Arc Bio System |
|---|---|
| Detector | 2998 PDA detector |
| Column | YMC-Pack Diol-200 |
| | 300 x 8.0 mmI.D; S-5 µm, 20 nm. DL.20S05 - 3008WT |
| Guard column | YMC-Guardpack: Diol-200 |
| | 30 x 8.0 mm I.D.; S-5 µm , 20 nm. |
| Mobile Phase | 25mM Sodium Phosphate, pH 6.8, 150 mM NaCl |
| Sample diluent | 25mM Sodium Phosphate, pH 6.8 |
| Sampler Temperature | 5 °C |
| Column Temperature | 30 °C |
| UV Detection | Wavelength: 280 nm |
| Flow Rate | 0.5 mL/min |
| Assay duration | 45 min |
| mAb RT | 18.8 min |

[Fig. 13] **Physical stability of adalimumab after 24 h incubation in simulated colonic environment with microbiota inoculation from two Crohn's Disease patients (Examples section I).**

[Fig. 14] **Biological activity of adalimumab after 24 h incubation in simulated colonic environment with microbiota inoculation from two Crohn's Disease patients (Examples section I).**

[Fig. 15] **Study design (Examples section J).**

**Sprague Dawley rats**

TNBS 80 mg/kg

TNBS period

Intrarectal Instillations or Subcutaneous Injections (250 µL every two Days)

D-5    D-3    D-1    D0    D1    D3    D4

Intrarectal Instillations or Subcutaneous Injections every two Days

**Groups of Sprague -Dawley rats (50 rats)**

1.  *10 TNBS rats + Vehicle (IR every 2 days start -D5 →D3)*
2.  *10 TNBS rats + Humira (15 mg/kg) IR (every 2 days start -D5 → D3)*
3.  *10 TNBS rats + Adalimumab (15 mg/kg) IR (every 2 days start -D5 → D3)*
4.  *10 TNBS rats + Humira (30 mg/kg) IR (every 2 days start -D5 → D3)*
5.  *10 TNBS rats + Humira (15 mg/kg) SC (every 2 days start -D5 → D3) = Positive control*

**Readouts**
1.  Body weight (daily)
2.  Mortality (daily)
3.  Collection of Colon, Liver, Spleen MLNs
4.  Wallace's score

[Fig. 16] **Representation of weight/length of the colon of colitic rats treated with different dosages of adalimumab (Examples section J-II, 2.3).**

[Fig. 17] **Representation of inflammation at macroscopic level, of colitic rats treated with adalimumab (Wallace score, Examples section J-II, 2.4).**

[Fig. 18] **Representation of inflammation at macroscopic level, of colitic rats treated with adalimumab + HPBCD (Wallace score, J-III, 2.4)**

[Fig. 19] **Representation of inflammation at histological level, treated with adalimumab + HPBCD (Ameho score, Examples section J-III, 2.5).**

[Fig. 20] **Quantity of Lcn-2 proteins (in pg/mg of total proteins), in the colon of colitic rats treated with adalimumab + HPBCD (Examples section J-IV, 2.1).**

[Fig. 21] **Levels of gene expression involved in the intestinal barrier reinforcement, at the mRNA level, in colitic rats treated with adalimumab + HPBCD (Examples section J-IV, 2.2).**

[Fig. 22] **Staining of proliferative IEC (green staining), in the colon of colitic rats treated with adalimumab + HPBCD (Examples section J-IV, 2.3.1).**

[Fig. 23] **Staining of apoptotic IECs (Red staining), in the colon of colitic rats treated with adalimumab + HPBCD (Examples section J-IV, 2.3.2).**

Europäisches
Patentamt

European
Patent Office

Office européen
des brevets

# EUROPEAN SEARCH REPORT

**Application Number**

EP 24 30 6112

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CN 108 201 140 A (INFINITUS CHINA CO LTD) 26 June 2018 (2018-06-26) * claim 1 * | 1-15 | INV. A61K9/28 A61K47/32 A61K47/36 A61K47/40 |
| Y | WO 2019/161408 A1 (SENSIENT COLORS LLC [US]) 22 August 2019 (2019-08-22) * claim 1 * | 1,7-10, 12-14 | |
| Y | US 2003/166508 A1 (ZHANG JUNSHOU [CN]) 4 September 2003 (2003-09-04) * claim 3 * | 2-4,11 | |
| Y | US 11 534 406 B2 (TILLOTTS PHARMA AG [CH]) 27 December 2022 (2022-12-27) * column 21, paragraph 1 * | 5,6,15 | |

**TECHNICAL FIELDS SEARCHED (IPC)**

A61K

The present search report has been drawn up for all claims

| Place of search | Date of completion of the search | Examiner |
|---|---|---|
| The Hague | 5 December 2024 | Friederich, Pierre |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 30 6112

This annex lists the patent family members relating to the patent documents cited in the above-mentioned European search report.
The members are as contained in the European Patent Office EDP file on
The European Patent Office is in no way liable for these particulars which are merely given for the purpose of information.

05-12-2024

| Patent document cited in search report | | Publication date | Patent family member(s) | | Publication date |
|---|---|---|---|---|---|
| CN 108201140 | A | 26-06-2018 | NONE | | |
| WO 2019161408 | A1 | 22-08-2019 | NONE | | |
| US 2003166508 | A1 | 04-09-2003 | AU | 8952101 A | 22-03-2002 |
| | | | CN | 1326784 A | 19-12-2001 |
| | | | US | 2003166508 A1 | 04-09-2003 |
| | | | WO | 0220037 A1 | 14-03-2002 |
| US 11534406 | B2 | 27-12-2022 | AR | 090898 A1 | 17-12-2014 |
| | | | AU | 2013255913 A1 | 13-11-2014 |
| | | | AU | 2013255914 A1 | 13-11-2014 |
| | | | AU | 2017210571 A1 | 24-08-2017 |
| | | | AU | 2017210577 A1 | 24-08-2017 |
| | | | BR | 112014026933 A2 | 27-06-2017 |
| | | | BR | 112014026935 A2 | 27-06-2017 |
| | | | BR | 122019022551 B1 | 19-07-2022 |
| | | | CA | 2871016 A1 | 07-11-2013 |
| | | | CA | 2871017 A1 | 07-11-2013 |
| | | | CA | 3052460 A1 | 07-11-2013 |
| | | | CA | 3080035 A1 | 07-11-2013 |
| | | | CL | 2014002795 A1 | 13-02-2015 |
| | | | CL | 2014002796 A1 | 06-02-2015 |
| | | | CN | 104271113 A | 07-01-2015 |
| | | | CN | 104302274 A | 21-01-2015 |
| | | | CN | 106983735 A | 28-07-2017 |
| | | | CN | 110200949 A | 06-09-2019 |
| | | | CN | 110237056 A | 17-09-2019 |
| | | | CO | 7141433 A2 | 12-12-2014 |
| | | | CO | 7151506 A2 | 29-12-2014 |
| | | | CR | 20140485 A | 19-01-2015 |
| | | | CR | 20140486 A | 19-01-2015 |
| | | | CR | 20190245 A | 29-07-2019 |
| | | | CR | 20190246 A | 03-09-2019 |
| | | | CU | 20140123 A7 | 26-02-2015 |
| | | | CY | 1120215 T1 | 12-12-2018 |
| | | | CY | 1120492 T1 | 10-07-2019 |
| | | | CY | 1121609 T1 | 29-05-2020 |
| | | | CY | 1122475 T1 | 27-01-2021 |
| | | | DK | 2659881 T3 | 05-02-2018 |
| | | | DK | 2844220 T3 | 08-04-2019 |
| | | | DK | 2844222 T3 | 09-12-2019 |
| | | | DK | 3189830 T3 | 16-07-2018 |
| | | | DK | 3278792 T3 | 06-05-2019 |
| | | | EA | 201491781 A1 | 30-12-2014 |
| | | | EA | 201491783 A1 | 30-12-2014 |

For more details about this annex : see Official Journal of the European Patent Office, No. 12/82

page 1 of 4

## ANNEX TO THE EUROPEAN SEARCH REPORT
## ON EUROPEAN PATENT APPLICATION NO.

EP 24 30 6112

This annex lists the patent family members relating to the patent documents cited in the above-mentioned European search report.
The members are as contained in the European Patent Office EDP file on
The European Patent Office is in no way liable for these particulars which are merely given for the purpose of information.

05-12-2024

| Patent document cited in search report | Publication date | Patent family member(s) | Publication date |
|---|---|---|---|
| | | EP 2659881 A1 | 06-11-2013 |
| | | EP 2844220 A1 | 11-03-2015 |
| | | EP 2844222 A1 | 11-03-2015 |
| | | EP 3187171 A1 | 05-07-2017 |
| | | EP 3189830 A1 | 12-07-2017 |
| | | EP 3278792 A1 | 07-02-2018 |
| | | ES 2655622 T3 | 20-02-2018 |
| | | ES 2673931 T3 | 26-06-2018 |
| | | ES 2714448 T3 | 28-05-2019 |
| | | ES 2720258 T3 | 19-07-2019 |
| | | ES 2761341 T3 | 19-05-2020 |
| | | ES 2987408 T3 | 14-11-2024 |
| | | GE P201706736 B | 25-09-2017 |
| | | GE P201706753 B | 25-10-2017 |
| | | GE P201706758 B | 25-10-2017 |
| | | GE P201706759 B | 25-10-2017 |
| | | HR P20180068 T1 | 23-03-2018 |
| | | HR P20180965 T1 | 10-08-2018 |
| | | HU E036187 T2 | 28-06-2018 |
| | | HU E037702 T2 | 28-09-2018 |
| | | HU E042833 T2 | 29-07-2019 |
| | | HU E043238 T2 | 28-08-2019 |
| | | HU E046628 T2 | 30-03-2020 |
| | | JO 3574 B1 | 05-07-2020 |
| | | JO P20200145 B1 | 17-09-2023 |
| | | JO P20200146 B1 | 17-09-2023 |
| | | JO P20200147 B1 | 17-09-2023 |
| | | JO P20200148 B1 | 17-09-2023 |
| | | JP 6621661 B2 | 18-12-2019 |
| | | JP 6621662 B2 | 18-12-2019 |
| | | JP 2015515964 A | 04-06-2015 |
| | | JP 2015515965 A | 04-06-2015 |
| | | KR 20150004910 A | 13-01-2015 |
| | | KR 20150004911 A | 13-01-2015 |
| | | KR 20190127984 A | 13-11-2019 |
| | | KR 20190127986 A | 13-11-2019 |
| | | KR 20200073296 A | 23-06-2020 |
| | | KR 20200075024 A | 25-06-2020 |
| | | LT 2659881 T | 12-02-2018 |
| | | LT 2844220 T | 10-04-2019 |
| | | LT 2844222 T | 27-12-2019 |
| | | LT 3189830 T | 10-07-2018 |
| | | LT 3278792 T | 10-05-2019 |
| | | ME 03364 B | 20-10-2019 |
| | | ME 03641 B | 20-07-2020 |
| | | MX 362529 B | 23-01-2019 |

EPO FORM P0459

For more details about this annex : see Official Journal of the European Patent Office, No. 12/82

page 2 of 4

## ANNEX TO THE EUROPEAN SEARCH REPORT
## ON EUROPEAN PATENT APPLICATION NO.

EP 24 30 6112

This annex lists the patent family members relating to the patent documents cited in the above-mentioned European search report.
The members are as contained in the European Patent Office EDP file on
The European Patent Office is in no way liable for these particulars which are merely given for the purpose of information.

05-12-2024

| Patent document cited in search report | Publication date | Patent family member(s) | | Publication date |
|---|---|---|---|---|
| | | MX 366677 B | | 19-07-2019 |
| | | MX 367127 B | | 06-08-2019 |
| | | MY 169088 A | | 15-02-2019 |
| | | MY 169161 A | | 19-02-2019 |
| | | MY 190392 A | | 20-04-2022 |
| | | NO 2659881 T3 | | 28-04-2018 |
| | | NZ 629260 A | | 27-01-2017 |
| | | NZ 629262 A | | 27-01-2017 |
| | | NZ 725409 A | | 25-05-2018 |
| | | PE 20142442 A1 | | 28-01-2015 |
| | | PE 20150129 A1 | | 13-02-2015 |
| | | PE 20190625 A1 | | 26-04-2019 |
| | | PH 12014502339 A1 | | 22-12-2014 |
| | | PH 12014502340 A1 | | 22-12-2014 |
| | | PL 2659881 T3 | | 30-05-2018 |
| | | PL 2844220 T3 | | 28-06-2019 |
| | | PL 2844222 T3 | | 31-03-2020 |
| | | PL 3189830 T3 | | 30-11-2018 |
| | | PL 3278792 T3 | | 30-09-2019 |
| | | PT 2659881 T | | 29-01-2018 |
| | | PT 2844220 T | | 27-03-2019 |
| | | PT 2844222 T | | 30-12-2019 |
| | | PT 3189830 T | | 19-10-2018 |
| | | PT 3278792 T | | 23-05-2019 |
| | | SG 11201406798W A | | 27-11-2014 |
| | | SG 11201406799X A | | 27-11-2014 |
| | | SI 2659881 T1 | | 30-03-2018 |
| | | SI 2844220 T1 | | 31-05-2019 |
| | | SI 2844222 T1 | | 28-02-2020 |
| | | SI 3189830 T1 | | 31-08-2018 |
| | | SI 3278792 T1 | | 28-06-2019 |
| | | TN 2014000441 A1 | | 30-03-2016 |
| | | TN 2014000442 A1 | | 30-03-2016 |
| | | TR 201809416 T4 | | 23-07-2018 |
| | | TR 201903569 T4 | | 22-04-2019 |
| | | TR 201905226 T4 | | 21-05-2019 |
| | | TW 201343202 A | | 01-11-2013 |
| | | TW 201720430 A | | 16-06-2017 |
| | | TW 202037370 A | | 16-10-2020 |
| | | US 2014056980 A1 | | 27-02-2014 |
| | | US 2015125525 A1 | | 07-05-2015 |
| | | US 2015132380 A1 | | 14-05-2015 |
| | | US 2017035698 A1 | | 09-02-2017 |
| | | US 2017266117 A1 | | 21-09-2017 |
| | | US 2020214987 A1 | | 09-07-2020 |
| | | UY 34772 A | | 29-11-2013 |

EPO FORM P0459

For more details about this annex : see Official Journal of the European Patent Office, No. 12/82

page 3 of 4

EP 4 674 409 A1

## ANNEX TO THE EUROPEAN SEARCH REPORT
## ON EUROPEAN PATENT APPLICATION NO.

EP 24 30 6112

This annex lists the patent family members relating to the patent documents cited in the above-mentioned European search report.
The members are as contained in the European Patent Office EDP file on
The European Patent Office is in no way liable for these particulars which are merely given for the purpose of information.

05-12-2024

| Patent document cited in search report | Publication date | Patent family member(s) | | Publication date |
|---|---|---|---|---|
| | | WO | 2013164315 A1 | 07-11-2013 |
| | | WO | 2013164316 A1 | 07-11-2013 |

EPO FORM P0459

For more details about this annex : see Official Journal of the European Patent Office, No. 12/82

page 4 of 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

Non-patent literature cited in the description

- **HAKOMORI, S.** ; **1964**. *J. Biol. Chem.*, vol. 55, 205
- **WALLACE JL** ; **KEENAN CM** ; **GALE D** ; **SHOUPE TS**. Exacerbation of experimental colitis by nonsteroidal anti-inflammatory drugs is not related to elevated leukotriene B4 synthesis.. *Gastroenterology*, January 1992, vol. 102 (1), 18-27
- **AMEHO C.K**. Prophylactic effect of dietary glutamine supplementation on interleukin 8 and tumor necrosis factor $\alpha$ production in trinitrobenzene sulfonic acid induced colitis.. *Gut.*, 1997, vol. 41, 487-493
- **HSIEH H et al.** *Toxicologic Pathology*, 2016
- **CHASSAING B et al.** *PlosOne*, 2012